(19) Europäisches Patentamt / European Patent Office / Office européen des brevets

(11) **EP 4 295 161 B1**

(12) **EUROPEAN PATENT SPECIFICATION**

(45) Date of publication and mention of the grant of the patent:
**23.07.2025 Bulletin 2025/30**

(21) Application number: **22710640.8**

(22) Date of filing: **16.02.2022**

(51) International Patent Classification (IPC):
**G01N 33/68** *(2006.01)*

(52) Cooperative Patent Classification (CPC):
**G01N 33/68; G01N 2800/326**

(86) International application number:
**PCT/FI2022/050099**

(87) International publication number:
**WO 2022/175596 (25.08.2022 Gazette 2022/34)**

(54) **USE OF GLYCOPROTEIN ACETYLS FOR DETERMINING THE RISK OF DEVELOPING ATRIAL FIBRILLATION AND/OR FLUTTER**

VERWENDUNG VON GLYKOPROTEINACETYLE ZUR BESTIMMUNG DES RISIKOS ZUR ENTWICKLUNG VON ATRIALES FLIMMERN UND FLATTERN

UTILISATION D'ACÉTYLES DE GLYCOPROTÉINES POUR DÉTERMINER LE RISQUE DE DÉVELOPPER UNE FIBRILLATION ET/OU UNE PALPITATION ATRIALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.02.2021 FI 20215176**

(43) Date of publication of application:
**27.12.2023 Bulletin 2023/52**

(73) Proprietor: **NIGHTINGALE HEALTH OYJ**
**00300 Helsinki (FI)**

(72) Inventors:
- **JULKUNEN, Heli**
  **00300 Helsinki (FI)**
- **WÜRTZ, Peter**
  **00300 Helsinki (FI)**

(74) Representative: **Papula Oy**
**P.O. Box 981**
**00101 Helsinki (FI)**

(56) References cited:
- RITCHIE SCOTT C. ET AL: "The Biomarker GlycA Is Associated with Chronic Inflammation and Predicts Long-Term Risk of Severe Infection", CELL SYSTEMS, vol. 1, no. 4, 1 October 2015 (2015-10-01), US, pages 293 - 301, XP055837905, ISSN: 2405-4712, Retrieved from the Internet <URL:https://www.cell.com/cell-systems/pdf/S2405-4712(15)00145-3.pdf> DOI: 10.1016/j.cels.2015.09.007
- JANG SUNYOUNG ET AL: "ABSTRACT 11299: The Novel Inflammatory Marker Glyc a and Incident Atrial Fibrillation in the Multi-ethnic Study of Atherosclerosis", CIRCULATION, 11 November 2019 (2019-11-11), pages 1 - 5, XP055912407, Retrieved from the Internet <URL:https://www.ahajournals.org/doi/10.1161/circ.140.suppl_1.11299#:~:text=2019%3B140%3AA11299-,Abstract,it%20a%20reliable%20inflammatory%20marker.> [retrieved on 20220413]

EP 4 295 161 B1

• VIVIANO ALESSANDRO ET AL: "Proteomics of the epicardial fat secretome and its role in post-operative atrial fibrillation", EUROPACE, vol. 20, no. 7, 1 July 2018 (2018-07-01), GB, pages 1201 - 1208, XP055912686, ISSN: 1099-5129, Retrieved from the Internet <URL:https://watermark. silverchair.com/eux113.pdf? token=AQECAHi208BE49Ooan9kkhW_Er cy7Dm3ZL_9Cf3qfKAc485ysgAAAulwggLeBgkq hkiG9w0BBwagggLPMlICywlBADCCAsQGCSqG SIb3DQEHATAeBglghkgBZQMEAS4wEQQMZhD C2sA6O4QVsgbOAgEQgllClf1o_JNjCOnRQZeW Eqo2kS2h155v8BvOya-4WCLvYhCOEQRoJ56kd dRgy6Rh6RM5areeUK0mylST3kA6nHnj9tsWkVb My> DOI: 10.1093/europace/eux113

• SAMUEL ADAMSSON ERYD ET AL: "Inflammation-sensitive proteins and risk of atrial fibrillation: a population-based cohort study", EUROPEAN JOURNAL OF EPIDEMIOLOGY, KLUWER ACADEMIC PUBLISHERS, DO, vol. 26, no. 6, 19 March 2011 (2011-03-19), pages 449 - 455, XP019916373, ISSN: 1573-7284, DOI: 10.1007/ S10654-011-9565-6

• MATTIOLI A V ET AL: "Atrial stunning, inflammation and nutritional status after cardioversion from atrial fibrillation", INTERNATIONAL JOURNAL OF CARDIOLOGY, ELSEVIER, AMSTERDAM, NL, vol. 129, no. 3, 13 October 2008 (2008-10-13), pages 344 - 347, XP025470679, ISSN: 0167-5273, [retrieved on 20080221], DOI: 10.1016/J.IJCARD.2007.07.103

**Description**

**TECHNICAL FIELD**

The present disclosure relates generally to methods for determining whether a subject is at risk of developing one or more of certain circulatory diseases.

**BACKGROUND**

Circulatory diseases are among the most common causes of hospitalization and death worldwide. Accurate identification of individuals at an elevated risk of developing circulatory diseases is important to support early treatment and prevention efforts. Current clinical tools focus primarily on predicting the risk of coronary artery disease and stroke. However, the risk prediction for many other types of circulatory diseases may also be beneficial. Such diseases include, for instance, chronic rheumatic heart disease, pulmonary heart disease, diseases of veins and lymphatic vessels, and other forms of heart disease such as nonrheumatic aortic valve disorders, cardiac arrest, and atrial fibrillation.

Various biomarkers may be useful for predicting whether an individual person is at an elevated risk of developing these circulatory diseases later in life. Such biomarkers may be measured from various biological samples, for example from blood samples or related biological fluids. Biomarkers predictive of the most severe presentations of these circulatory diseases, causing hospitalization or even death, could provide support for better targeted screening, earlier diagnosis and preventative treatment.

Jang et al., Abstract 11299: The novel inflammatory marker Glyc a and incident atrial fibrillation in the multi-ethnic study of atherosclerosis, Circulation 11 November 2019, pages 1-5 describes the association between GlycA and atrial fibrillation in a multi-ethnic cohort.

Viviano et al., Proteomics of the epicardial fat secretome and its role in post-operative atrial fibrillation, Europace 2018, 20, 1201-1208 describes differentially expressed proteins in patients who later develop post-operative atrial fibrillation.

Adamsson Eryd et al., Inflammation-sensitive proteins and risk of atrial fibrillation: a population-based cohort study, European Journal of Epidemiology 2011, 26, 449-455 explores the association of plasma levels of inflammation-sensitive proteins and incidence of atrial fibrillation in a population-based cohort.

Mattioli et al., Atrial stunning, inflammation and nutritional status after cardioversion from atrial fibrillation, International Journal of Cardiology 2008, 129, 344-347 evaluates the relationship between nutritional status and atrial stunning after conversion of atrial fibrillation.

**SUMMARY**

A method for determining whether a subject is at risk of developing a circulatory disease is disclosed. The method comprisees determining in a biological sample obtained from the subject a quantitative value of at least one biomarker of the following:

- albumin,
- glycoprotein acetyls,
- a ratio of linoleic acid to total fatty acids,
- a ratio of omega-6 fatty acids to total fatty acids,
- a ratio of saturated fatty acids to total fatty acids,
- fatty acid degree of unsaturation,
- linoleic acid,
- omega-6 fatty acids,
- triglycerides in intermediate-density lipoprotein (IDL),
- triglycerides in low-density lipoprotein (LDL),
- acetate,
- lactate,
- pyruvate,
- acetoacetate,
- alanine,
- glutamine,
- glycine,
- histidine,
- phenylalanine,

- tyrosine; and

comparing the quantitative value(s) of the at least one biomarker to a control sample or to a control value;
wherein an increase or a decrease in the quantitative value(s) of at least one biomarker, when compared to the control sample or to the control value, is/are indicative of the subject having an increased risk of developing the circulatory disease;
wherein the circulatory disease comprises or is atrial fibrillation and/or flutter; and
wherein the at least one biomarker comprises or is glycoprotein acetyls, wherein glycoprotein acetyls refers to a nuclear magnetic resonance spectroscopy signal that represents the abundance of circulating glycated proteins.

The subject may be generally healthy, i.e. known not to suffer from a circulatory disease, or the subject may have an already existing form of a circulatory disease and the risk of developing another or recurrent circulatory disease may be determined.

## BRIEF DESCRIPTION OF THE DRAWINGS

The accompanying drawings, which are included to provide a further understanding of the embodiments and constitute a part of this specification, illustrate various embodiments. In the drawings:

**Figure 1a** shows the relation of baseline concentrations of 20 blood biomarkers to future development of Certain Circulatory Diseases (here defined as a composite endpoint of the following circulatory disease ICD-10 diagnoses: I08, I10, I26, I27, I31, I35, I42, I44, I45, I46, I48, I71, I74, I77, I80 and I89; here termed "Certain Circulatory Diseases"), when the biomarker concentrations are analysed in absolute concentrations and in quintiles of biomarker concentrations. Results are based on plasma samples from approximately 115 000 generally healthy individuals from the UK Biobank.

**Figure 1b** shows the cumulative risk for "Certain Circulatory Diseases" during follow-up for the lowest, middle, and highest quintiles of the 20 blood biomarker concentrations.

**Figure 2a** shows the relation of baseline biomarker levels to the future development of 16 specific circulatory diseases (defined by ICD-10 3-character diagnoses) in the form of a heatmap. The results demonstrate that the specific circulatory diseases defined by 3-character ICD-10 codes all have highly similar associations with a broad panel of biomarkers measured by NMR spectroscopy of plasma samples from generally healthy humans.

**Figure 2b** shows the consistency of the biomarker associations with specific circulatory diseases (defined by ICD-10 3-character diagnoses), in comparison to direction of the association with "Certain Circulatory Diseases".

**Figures 3a, 3b, 3c, 3d, 3e, 3f, 3g, and 3h** show the relation of baseline biomarker levels to the future development of the 16 specific circulatory diseases, in the form of foresplots of the hazard ratios for incident disease onset.

**Figure 4** shows the relation of baseline concentrations of the 20 blood biomarkers to the risk of death from "Certain Circulatory Diseases" as compared to the risk of future hospitalisation and/or death from "Certain Circulatory Diseases". The results demonstrate that the biomarkers are often stronger predictors for fatal events than for hospitalisation.

**Figure 5** shows an example of the relation of multi-biomarker scores to the risk of "Certain Circulatory Diseases". Selected examples of multi-biomarker scores are shown to illustrate the improved prediction attained by multi-biomarker scores as compared to individual biomarkers.

**Figure 6a** shows an intended use case for a multi-biomarker score to predict the risk for developing multiple valve diseases among initially healthy humans.

**Figure 6b** shows that the prediction of the risk for developing multiple valve diseases works effectively for people with different demographics and when adjusting for common risk factors, with substantially stronger results for short term risk prediction.

**Figure 7a** shows an intended use case for a multi-biomarker score to predict the risk for developing pulmonary embolism among initially healthy humans.

**Figure 7b** shows that the prediction of the risk for developing pulmonary embolism works effectively for people with different demographics and when adjusting for common risk factors, with substantially stronger results for short term risk prediction.

**Figure 8a** shows an intended use case for a multi-biomarker score to predict the risk for developing nonrheumatic aortic valve disorders among initially healthy humans.

**Figure 8b** shows that the prediction of the risk for developing nonrheumatic aortic valve disorders works effectively for people with different demographics and when adjusting for common risk factors, with substantially stronger results for short term risk prediction.

**Figure 9a** shows an intended use case for a multi-biomarker score to predict the risk for developing atrial fibrillation and flutter among initially healthy humans.

**Figure 9b** shows that the prediction of the risk for developing atrial fibrillation and flutter works effectively for people with different demographics and when adjusting for common risk factors, with substantially stronger results for short term risk prediction.

## DETAILED DESCRIPTION

A method for determining whether a subject is at risk of developing a circulatory disease is disclosed.

The method comprises determining in a biological sample obtained from the subject a quantitative value of at least one biomarker of the following:

- albumin,
- glycoprotein acetyls,
- a ratio of linoleic acid to total fatty acids,
- a ratio of omega-6 fatty acids to total fatty acids,
- a ratio of saturated fatty acids to total fatty acids,
- fatty acid degree of unsaturation,
- linoleic acid,
- omega-6 fatty acids,
- triglycerides in intermediate-density lipoprotein (IDL),
- triglycerides in low-density lipoprotein (LDL),
- acetate,
- lactate,
- pyruvate,
- acetoacetate,
- alanine,
- glutamine,
- glycine,
- histidine,
- phenylalanine,
- tyrosine; and

and comparing the quantitative value(s) of the at least one biomarker to a control sample or to a control value; wherein an increase or a decrease in the quantitative value(s) of the at least one biomarker, when compared to the control sample or to the control value, is/are indicative of the subject having an increased risk of developing the circulatory disease.

The subject may be generally healthy, i.e. known not to suffer from a circulatory disease, or the subject may have or may have had an already existing form of a circulatory disease (the same or different circulatory disease). In the latter case, the risk of developing another or a recurrent circulatory disease may be determined. In other words, the risk of developing a circulatory disease different from the one the subject may already have (or may already have had) may be determined, and/or the risk of developing a recurrent form of the same circulatory disease the subject may already have (or may already have had) may be determined.

Various blood biomarkers may be useful for predicting whether an individual person is at elevated risk of developing the circulatory disease. Such biomarkers may be measured from biological samples, for example from blood samples or related biological fluids.

Biomarkers predictive of hospitalization from the circulatory disease could help to enable more effective screening and better targeted preventative treatment.

In an embodiment, the method comprises determining a quantitative value of albumin.

The method comprises determining a quantitative value of glycoprotein acetyls.

In an embodiment, the method comprises determining a quantitative value of ratio of linoleic acid to total fatty acids.

In an embodiment, the method comprises determining a quantitative value of ratio of omega-6 fatty acids to total fatty acids.

In an embodiment, the method comprises determining a quantitative value of ratio of saturated fatty acids to total fatty acids.

In an embodiment, the method comprises determining a quantitative value of fatty acid degree of unsaturation.

In an embodiment, the method comprises determining a quantitative value of linoleic acid.

In an embodiment, the method comprises determining a quantitative value of omega-6 fatty acids.

In an embodiment, the method comprises determining a quantitative value of triglycerides in intermediate-density lipoprotein (IDL).

In an embodiment, the method comprises determining a quantitative value of triglycerides in low-density lipoprotein (LDL) .

In an embodiment, the method comprises determining a quantitative value of acetate.

In an embodiment, the method comprises determining a quantitative value of lactate.

In an embodiment, the method comprises determining a quantitative value of pyruvate.

In an embodiment, the method comprises determining a quantitative value of acetoacetate.

In an embodiment, the method comprises determining a quantitative value of alanine.

In an embodiment, the method comprises determining a quantitative value of glutamine.

In an embodiment, the method comprises determining a quantitative value of glycine.

In an embodiment, the method comprises determining a quantitative value of histidine.

In an embodiment, the method comprises determining a quantitative value of phenylalanine.

In an embodiment, the method comprises determining a quantitative value of tyrosine.

The metabolic biomarker (s) described in this specification have been found to be significantly different, i.e. their quantitative values (such as amount and/or concentration) have been found to be significantly higher or lower, for subjects who later developed a certain circulatory disease. The biomarkers may be detected and quantified from blood, serum, or plasma, dry blood spots, or other suitable biological sample, and may be used to determine the risk of developing the circulatory disease, either alone or in combination with other biomarkers.

Furthermore, the biomarker(s) may significantly improve the possibility of identifying subjects at risk for developing the circulatory disease, even in combination with and/or when accounting for established risk factors that may currently be used for screening and risk prediction, such as age, sex, smoking status, alcohol use, body mass index (BMI), waist circumference, physical activity, diet, blood pressure, blood cholesterol, family history, genetic risk, and/or prior medical history of circulatory diseases and/or other comorbidities. The biomarkers described in this specification, alone or as a risk score (such as a multi-biomarker score), hazard ratio, odds ratio, and/or predicted absolute or relative risk, or in combination with other risk factors and tests, may improve prediction or even replace the need for other tests or measures. This may include improving prediction accuracy by complementing the predictive information from other risk factors and tests, or by replacing the need for other analyses, such as ECG (electrocardiogram), coronary calcium scan and/or blood tests for, for instance, LDL, HDL and/or total cholesterol, triglycerides, inflammatory protein biomarkers such as CRP (C-reactive protein), NT-proBNP (N-terminal (NT)-pro hormone BNP) and/or hs-cTnT (high-sensitivity cardiac troponin T) and/or genetic scores on risk for circulatory diseases. The biomarkers or the risk score, hazard ratio, odds ratio, and/or predicted absolute or relative risk according to one or more embodiments described in this specification may thus allow for efficiently assessing the risk for future development of the circulatory disease, also in conditions in which other risk factor measures are not as feasible.

The method may comprise determining in the biological sample quantitative values of a plurality of the biomarkers, such as two, three, four, five or more biomarkers. For example, the plurality of the biomarkers may comprise 2, 3, 4, 5, 6, 7, 8, 9, 10, 11, 12, 13, 14, 15, 16, 17, 18, 19 or 20 (i.e. at least 2, at least 3, at least 4, at least 5, at least 6, at least 7, at least 8, at least 9, at least 10, at least 11, at least 12, at least 13, at least 14, at least 15, at least 16, at least 17, at least 18, at least 19) or all of the biomarkers. The term "plurality of the biomarkers" may thus, within this specification, be understood as referring to any number (above one) of the biomarkers. The term "plurality of the biomarkers" may thus be understood as referring to any number (above one) and/or combination or subset of the biomarkers described in this specification. Determining the plurality of the biomarkers may increase the accuracy of the prediction of whether the subject is at risk of developing the circulatory disease. In general, it may be that the higher the number of the biomarkers, the more accurate or predictive the method. However, even a single biomarker described in this specification may allow for or assist in determining whether the subject is at risk of developing the circulatory disease. The plurality of the biomarkers may be measured from the same biological sample or from separate biological samples and using the same analytical method or different analytical methods. In an embodiment, the plurality of biomarkers may be a panel of a plurality of biomarkers.

In the context of this specification, the wording "comparing the quantitative value(s) of the biomarker(s) to a control sample or to a control value(s)" may be understood, as a skilled person would, as referring to comparing the quantitative value or values of the biomarker or biomarkers, to a control sample or to a control value(s) either individually or as a plurality of biomarkers (e.g. when a risk score is calculated from the quantitative values of a plurality of biomarkers), depending e.g. on whether the quantitative value of a single (individual) biomarker or the quantitative values of a plurality of biomarkers are determined.

In an embodiment, the method may comprise determining in the biological sample obtained from the subject a quantitative value/values of the following biomarkers:

- albumin,

- glycoprotein acetyls,
- a ratio of linoleic acid to total fatty acids,
- a ratio of omega-6 fatty acids to total fatty acids,
- a ratio of saturated fatty acids to total fatty acids,
- fatty acid degree of unsaturation,
- linoleic acid,
- omega-6 fatty acids,
- triglycerides in intermediate-density lipoprotein (IDL),
- triglycerides in low-density lipoprotein (LDL),
- acetate,
- lactate,
- pyruvate,
- acetoacetate,
- alanine,
- glutamine,
- glycine,
- histidine,
- phenylalanine,
- tyrosine; and

comparing the quantitative value(s) of the biomarkers to a control sample or to a control value(s);
wherein an increase or a decrease in the quantitative value(s) of the biomarkers, when compared to the control sample or to the control value, is/are indicative of the subject having an increased risk of developing the circulatory disease.

The at least one biomarker comprises or is glycoprotein acetyls. The method may further comprise determining a quantitative value of at least one of the other biomarkers described in this specification.

The subject may be human. The human may be healthy or have an existing disease, such as an existing circulatory disease. Specifically, the human may have an already existing form of a circulatory disease, and the risk for developing recurrent and/or more severe form(s) of said disease and/or other circulatory disease(s) may be determined and/or calculated. The subject may, additionally or alternatively, be an animal, such as a mammal, for example, a non-human primate, a dog, a cat, a horse, or a rodent.

In the context of this specification, the term "biomarker" may refer to a biomarker, for example a chemical or molecular marker, that may be found to be associated with a disease or a condition or the risk of having or developing thereof. It does not necessarily refer to a biomarker that would be statistically fully validated as having a specific effectiveness in a clinical setting. The biomarker may be a metabolite, a compound, a lipid, a protein, a moiety, a functional group, a composition, a combination of two or more metabolites and/or compounds, a (measurable or measured) quantity thereof, a ratio or other value derived thereof, or in principle any measurement reflecting a chemical and/or biological component that may be found to be associated with a disease or condition or the risk of having or developing thereof. The biomarkers and any combinations thereof, optionally in combination with further analyses and/or measures, may be used to measure a biological process indicative of the risk for developing the circulatory disease, such as multiple valve disease, pulmonary embolism, nonrheumatic aortic valve disorders, or atrial fibrillation and/or flutter.

The disease may refer to a category of circulatory diseases or to a specific disease in this category. In the context of this specification, the term "a circulatory disease" or "a certain circulatory disease" may be understood as referring to one or more of the diseases of the circulatory system of the following: a multiple valve disease (ICD-10 diagnosis code I08); essential (primary) hypertension (I10); a pulmonary embolism (I26); other pulmonary heart disease (I27); other disease of pericardium (I31); a nonrheumatic aortic valve disorder (I35); a cardiomyopathy (I42); an atrioventricular and/or left bundle-branch block (I44); other conduction disorder (I45); a cardiac arrest (I46); atrial fibrillation and/or flutter (I48); an aortic aneurysm and/or dissection (I71); an arterial embolism and/or thrombosis (I74); other disorder of arteries and/or arterioles (I77); a phlebitis and/or thrombophlebitis (I80); and/or other noninfective disorder of lymphatic vessels and/or lymph nodes (I89). It may be an acute condition or a chronic condition. The chronic condition may, in the context of this specification, be understood as persistent or otherwise long-lasting in its effects and/or a disease that comes with time. The signs and symptoms of circulatory diseases may vary from mild to severe or disabling or potentially life-threatening, depending on factors such as age and/or overall health of the subject.

In an embodiment, the method is a method for determining whether the subject is at risk of developing or dying from the circulatory disease within (the following) three years.

The biomarker associations may be similar for the different circulatory diseases. Therefore, the same individual biomarkers and combinations of biomarkers may be extended to also predict the risk for specific circulatory diseases. Examples of such specific circulatory diseases include multiple valve diseases, pulmonary embolism, nonrheumatic aortic valve disorders, atrial fibrillation and/or flutter.

Circulatory diseases described in this specification may be classified as follows. "ICD-10" may be understood as referring to the International Statistical Classification of Diseases and Related Health Problems 10th Revision (ICD-10) - WHO Version for 2019. Similar diseases classified or diagnosed by other disease classification systems than ICD-10, such as ICD-9 or ICD-11, may also apply.

The term "Certain Circulatory Diseases" may be understood as referring to any incident occurrence of ICD-10 diagnoses I08, I10, I26, I27, I31, I35, I42, I44, I45, I46, I48, I71, I74, I77, I80 or I89.

Specific circulatory diseases may be understood as referring to diseases and classified within the 3-character ICD-10 diagnoses for circulatory diseases (I08, I10, I26, I27, I31, I35, I42, I44, I45, I46, I48, I71, I74, I77, I80, 189).

In an embodiment, the circulatory disease is a specific disease, such as a disease defined by one or more ICD-10 3-character code diagnoses described in this specification.

In an embodiment, the circulatory disease is a disease within at least one of the following ICD-10 3-character diagnoses:

- I48: Atrial fibrillation and flutter

In an embodiment, the circulatory disease may comprise or be death from a circulatory disease, such as any circulatory disease described in this specification or a disease denoted by one or more of the ICD-10 codes listed above.

In an embodiment, the circulatory disease may comprise or be atrial fibrillation and/or flutter (148).

The circulatory disease comprises or is atrial fibrillation and/or flutter.

The method may further comprise determining whether the subject is at risk of developing the circulatory disease using a risk score, hazard ratio, and/or predicted absolute or relative risk calculated on the basis of the quantitative value(s) of the at least one biomarker or of the plurality of the biomarkers.

An increase or a decrease in the risk score, hazard ratio, and/or predicted absolute risk and/or relative risk may be indicative of the subject having an increased risk of developing the circulatory disease.

The risk score and/or hazard ratio and/or predicted absolute risk or relative risk may be calculated based on any plurality, combination or subset of biomarkers described in this specification.

The risk score and/or hazard ratio and/or predicted absolute risk or relative risk may be calculated e.g. as shown in the Examples below. For example, the plurality of biomarkers measured using a suitable method, for example with NMR spectroscopy, may be combined using regression algorithms and multivariate analyses and/or using machine learning analysis. Before regression analysis or machine learning, any missing values in the biomarkers may be imputed with the mean value of each biomarker for the dataset. A number of biomarkers, for example five, that may be considered most associated with the onset of the disease or condition may be selected for use in the prediction model. Other modelling approaches may be used to calculate a risk score and/or hazard ratio and/or predicted absolute risk or relative risk based on a combination or subset of individual biomarkers, i.e. a plurality of the biomarkers.

The risk score may be calculated e.g. as a weighted sum of individual biomarkers, i.e. a plurality of the biomarkers. The weighted sum may be e.g. in the form of a multi-biomarker score defined as $\sum_i [(\beta_i * c_i)] + \beta_0$; where $i$ is the index of summation over individual biomarkers, $\beta_i$ is the weighted coefficient attributed to biomarker $i$, $c_i$ is the blood concentration of biomarker $i$, and $\beta_0$ is an intercept term.

For example, the risk score can be defined as: $\beta_1$ concentration (glycoprotein acetyls) + $\beta_2$* concentration (ratio of saturated fatty acids to total fatty acids) + $\beta_3$* concentration (albumin) + $\beta_0$, where $\beta_1$, $\beta_2$, $\beta_3$ are multipliers for each biomarker according to the association magnitude with the risk of the circulatory disease and $\beta_0$ is an intercept term. As a skilled person will understand, the biomarkers mentioned in this example may be replaced by any other biomarker(s) described in this specification. In general, the more biomarkers are included in the risk score, the stronger the predictive performance may become. When additional biomarkers are included in the risk score, the $\beta_i$ weights may change for all biomarkers according to the optimal combination for the prediction of the circulatory disease.

The risk score, hazard ratio, odds ratio, and/or predicted relative risk and/or absolute risk may be calculated on the basis of at least one further measure, for example a characteristic of the subject. Such characteristics may be determined prior to, simultaneously, or after the biological sample is obtained from the subject. As a skilled person will understand, some of the characteristics may be information collected e.g. using a questionnaire or clinical data collected earlier. Some of the characteristics may be determined (or may have been determined) by biochemical or clinical diagnostic measurements and/or medical diagnosis. Such characteristics could include, for example, one or more of age, height, weight, body mass index, race or ethnic group, smoking, and/or family history of circulatory diseases.

The risk prediction for the circulatory disease based on one or more of the biomarkers can be used to guide preventative efforts, such as non-smoking awareness, healthy diet, regular exercise, physical activity, and/or clinical screening frequency, and/or pharmacological treatment decisions, in particular lipid-lowering and antihypertensive medications. For example, the information of the future risk for the circulatory disease can be used for guiding treatment with, for instance, anticoagulants, angiotensin-converting enzyme (ACE) inhibitors, angiotensin II receptor blockers (ARBs), beta-blockers, antiplatelet agents, calcium channel blokers, diuretics, digitalis medication, nitrates and/or cholesterol-lowering medications such as statins, ezetimibe and fibrates.

In the context of this specification, the term "albumin" may be understood as referring to serum albumin (often referred to as blood albumin). It is an albumin found in vertebrate blood. Albumin is a globular, water-soluble, un-glycosylated serum protein of approximate molecular weight of 65,000 Daltons. The measurement of albumin using NMR is described e.g. in publications by Kettunen et al., 2012, Nature Genetics 44, 269-276; Soininen et al., 2015, Circulation: Cardiovascular Genetics 8, 212-206 (DOI: 10.1161/CIRCGENETICS.114.000216). Albumin may also be measured by various other methods, for example by clinical chemistry analyzers. Examples of such methods may include e.g. dye-binding methods such as bromocresol green and bromocresol purple.

In the context of this specification, the term "glycoprotein acetyls", "glycoprotein acetylation", or "GlycA" refers to a nuclear magnetic resonance spectroscopy (NMR) signal that represents the abundance of circulating glycated proteins, i.e. N-acetylated glycoproteins. Glycoprotein acetyls may include signals from a plurality of different glycoproteins, including e.g. alpha-1-acid glycoprotein, alpha-1 antitrypsin, haptoglobin, transferrin, and/or alpha-1 antichymotrypsin. In the scientific literature on cardiometabolic biomarkers, the terms "glycoprotein acetyls" or "GlycA" may commonly refer to the NMR signal of circulating glycated proteins (e.g. Ritchie et al, Cell Systems 2015 1(4):293-301; Connelly et al, J Transl Med. 2017;15(1):219). Glycoprotein acetyls and a method for measuring them is described e.g. in Kettunen et al., 2018, Circ Genom Precis Med. 11:e002234 and Soininen et al., 2009, Analyst 134, 1781-1785. There may be benefits of using the NMR signal of glycoprotein acetyls for risk prediction above measurement of the individual proteins contributing to the NMR signal, for instance better analytical accuracy and stability over time, as well as lower costs of the measurement and the possibility to measure the NMR signal simultaneously with many other biomarkers.

In the context of this specification, the term "omega-6 fatty acids" may refer to total omega-6 fatty acids, i.e. the total omega-6 fatty acid amounts and/or concentrations, i.e. the sum of the amounts and/or concentrations of different omega-6 fatty acids. Omega-6 fatty acids are polyunsaturated fatty acids. In omega-6 fatty acids, the last double bond in the fatty acid chain is the sixth bond counting from the methyl end.

In one embodiment, the omega-6 fatty acid may be linoleic acid. Linoleic acid (18:2ω-6) is the most abundant type of omega-6 fatty acids, and may therefore be considered as a good approximation for total omega-6 fatty acids for risk prediction of the circulatory disease.

In the context of this specification, the term "saturated fatty acids" (SFAs) may refer to total saturated fatty acids. Saturated fatty acids may be or comprise fatty acids which have no double bonds in their structure. Palmitic acid (16:0) and stearic acid (18:0) are examples of abundant SFAs in human serum.

For all fatty acid measures, including omega-6, linoleic acid and/or saturated fatty acids, the fatty acid measures may include blood (or serum/plasma) free fatty acids, bound fatty acids and esterified fatty acids. Esterified fatty acids may, for example, be esterified to glycerol as in triglycerides, diglycerides, monoglycerides, or phosphoglycerides, or to cholesterol as in cholesterol esters.

In the context of this specification, the term "fatty acid degree of unsaturation" or "unsaturation" may be understood as referring to the number of double bonds in total fatty acids, for example the average number of double bonds in total fatty acids.

In the context of this specification, the term "IDL" refers to intermediate-density lipoprotein.

In the context of this specification, the term "LDL" refers to low-density lipoprotein.

In the context of this specification, the phrase "low-density lipoprotein (LDL) triglycerides", "intermediate-density lipoprotein (IDL) triglycerides", "triglycerides in LDL (low-density lipoprotein)", or "triglycerides in IDL (intermediate-density lipoprotein)", may be understood as referring to total triglyceride concentration in said lipoprotein class or subfraction.

In the context of this specification, the term "acetate" may refer to the acetate molecule and/or acetic acid, for example in blood, plasma or serum or related biofluids.

In the context of this specification, the term "citrate" may refer to the citrate molecule and/or citric acid, for example in blood, plasma or serum or related biofluids.

In the context of this specification, the term "lactate" may refer to the lactate molecule and/or lactic acid, for example in blood, plasma or serum or related biofluids.

In the context of this specification, the term "pyruvate" may refer to the pyruvate molecule and/or pyruvic acid, for example in blood, plasma or serum or related biofluids.

In the context of this specification, the term "acetoacetate" may refer to the acetoacetate molecule and/or acetoacetic acid, for example in blood, plasma or serum or related biofluids.

In the context of this specification, the term "alanine" may refer to the alanine amino acid, for example in blood, plasma or serum or related biofluids.

In the context of this specification, the term "glutamine" may refer to the glutamine amino acid, for example in blood, plasma or serum or related biofluids.

In the context of this specification, the term "glycine" may refer to the glycine amino acid, for example in blood, plasma or serum or related biofluids.

In the context of this specification, the term "histidine" may refer to the histidine amino acid, for example in blood, plasma or serum or related biofluids.

In the context of this specification, the term "phenylalanine" may refer to the phenylalanine amino acid, for example in blood, plasma or serum or related biofluids.

In the context of this specification, the term "tyrosine" may refer to the tyrosine amino acid, for example in blood, plasma or serum or related biofluids.

In the context of this specification, the term "quantitative value" may refer to any quantitative value characterizing the amount and/or concentration of a biomarker. For example, it may be or reflect the amount or concentration of the biomarker in the biological sample, or it may be or reflect a signal derived from nuclear magnetic resonance spectroscopy (NMR) or other method suitable for detecting the biomarker in a quantitative manner. Such a signal may be indicative of or may correlate with the amount or concentration of the biomarker. It may also be a quantitative value calculated from one or more signals derived from NMR measurements or from other measurements. Quantitative values may, additionally or alternatively, be measured using a variety of techniques. Such methods may include mass spectrometry (MS), gas chromatography combined with MS, high performance liquid chromatography alone or combined with MS, immunoturbidimetric measurements, ultracentrifugation, ion mobility, enzymatic analyses, colorimetric or fluorometric analyses, immunoblot analysis, immunohistochemical methods (e.g. *in situ* methods based on antibody detection of metabolites), and immunoassays (e.g. ELISA). Examples of various methods are set out below. The method used to determine the quantitative value(s) in the subject may be the same method that is used to determine the quantitative value(s) in a control subject/control subjects or in a control sample/control samples.

The quantitative value, or the initial quantitative value, of the at least one biomarker, or the plurality of the biomarkers, may be measured using nuclear magnetic resonance (NMR) spectroscopy, for example $^1$H-NMR. The at least one additional biomarker, or the plurality of the additional biomarkers, may also be measured using NMR. NMR may provide a particularly efficient and fast way to measure biomarkers, including a large number of biomarkers simultaneously, and can provide quantitative values for them. NMR also typically requires very little sample pre-treatment or preparation. The biomarkers measured with NMR can effectively be measured for large amounts of samples using an assay for blood (serum or plasma) NMR metabolomics previously published by Soininen et al., 2015, Circulation: Cardiovascular Genetics 8, 212-206 (DOI: 10.1161/CIRCGENETICS.114.000216); Soininen et al., 2009, Analyst 134, 1781-1785; and Würtz et al., 2017, American Journal of Epidemiology 186 (9), 1084-1096 (DOI: 10.1093/aje/kwx016). This provides data on 250 biomarkers per sample as described in detail in the above scientific papers.

In an embodiment, the (initial) quantitative value of the at least one biomarker is/are measured using nuclear magnetic resonance spectroscopy.

However, quantitative values for various biomarkers described in this specification may also be performed by techniques other than NMR. For example, mass spectrometry (MS), enzymatic methods, antibody-based detection methods, or other biochemical or chemical methods may be contemplated, depending on the biomarker.

E.g. monounsaturated fatty acids, saturated fatty acids, and omega-6 fatty acids can be quantified (i.e. their quantitative values may be determined) by serum total fatty acid composition using gas chromatography (for example, as described in Jula et al., 2005, Arterioscler Thromb Vasc Biol 25, 2152-2159).

In the context of this specification, the term "sample" or "biological sample" may refer to any biological sample obtained from a subject or a group or population of subjects. The sample may be fresh, frozen, or dry.

The biological sample may comprise or be, for example, a blood sample, a plasma sample, a serum sample, or a sample derived therefrom. The biological sample may be, for example, a fasting blood sample, a fasting plasma sample, a fasting serum sample, or a fraction obtainable therefrom. However, the biological sample does not necessarily have to be a fasting sample. The blood sample may be a venous blood sample.

The blood sample may be a dry blood sample. The dry blood sample may be a dried whole blood sample, a dried plasma sample, a dried serum sample, or a dried sample derived therefrom.

The biological sample may be obtained from the subject prior to determining the quantitative value of the at least one biomarker. Taking a blood sample or a tissue sample of a subject or patient is a part of normal clinical practice. The collected blood or tissue sample can be prepared and serum or plasma can be separated using techniques well known to a skilled person. Methods for separating one or more fractions from biological samples, such as blood samples or tissue samples, are also available to a skilled person. The term "fraction" may, in the context of this specification, also refer to a portion or a component of the biological sample separated according to one or more physical properties, for instance solubility, hydrophilicity or hydrophobicity, density, or molecular size.

In the context of this specification, the term "control sample" may refer to a sample obtained from a subject and known not to suffer from the disease or condition or not being at risk of having or developing the disease or condition. The control sample may be matched. In an embodiment, the control sample may be a biological sample from a healthy individual or a generalized population of healthy individuals. The term "control value" may be understood as a value obtainable from the control sample and/or a quantitative value derivable therefrom. For example, it may be possible to calculate a threshold value from control samples and/or control values, above or below which the risk of developing the disease or condition is elevated. In other words, a value higher or lower (depending on the biomarker, risk score, hazard ratio, and/or predicted absolute risk or relative risk) than the threshold value may be indicative of the subject having an increased risk of developing the disease or condition.

An increase or a decrease in the quantitative value(s) of the at least one biomarker, or the plurality of the biomarkers, when compared to the control sample or to the control value, may be indicative of the subject having an increased risk of having or developing the disease or condition. Whether an increase or a decrease is indicative of the subject having an increased risk of developing the disease or condition, may depend on the biomarker.

A 1.2-fold, 1.5-fold, or for example 2-fold, or 3-fold, increase or a decrease in the quantitative value(s) of the at least one biomarker (or in an individual biomarker of the plurality of the biomarkers) when compared to the control sample or to the control value, may be indicative of the subject having an increased risk of developing the disease or condition.

In an embodiment, a decrease in the quantitative value of albumin, when compared to the control sample or to the control value, may be indicative of the subject having an increased risk of developing the circulatory disease, such as a multiple valve disease, a pulmonary embolism, a nonrheumatic aortic valve disorder, atrial fibrillation and/or flutter.

In an embodiment, an increase in the quantitative value of glycoprotein acetyls, when compared to the control sample or to the control value, may be indicative of the subject having an increased risk of developing the circulatory disease, such as a multiple valve disease, a pulmonary embolism, a nonrheumatic aortic valve disorder, atrial fibrillation and/or flutter.

In an embodiment, a decrease in the quantitative value of the ratio of linoleic acid to total fatty acids, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, a decrease in the quantitative value of the ratio of omega-6 fatty acids and/or of linoleic acid to total fatty acids, when compared to the control sample or to the control value, may be indicative of the subject having an increased risk of developing the circulatory disease, such a multiple valve disease, pulmonary embolism, a nonrheumatic aortic valve disorder, atrial fibrillation and/or flutter.

In an embodiment, an increase in the quantitative value of the ratio of saturated fatty acids to total fatty acids, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, a decrease in the quantitative value of fatty acid degree of unsaturation, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, a decrease in the quantitative value of linoleic acid, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, a decrease in the quantitative value of omega-6 fatty acids, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, an increase in the quantitative value of triglycerides in intermediate-density lipoprotein (IDL), when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, an increase in the quantitative value of triglycerides in low-density lipoprotein (LDL), when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, a decrease in the quantitative value of acetate, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, an increase in the quantitative value of lactate, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, an increase in the quantitative value of pyruvate, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, an increase in the quantitative value of acetoacetate, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, an increase in the quantitative value of alanine, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, a decrease in the quantitative value of glutamine, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, a decrease in the quantitative value of glycine, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, a decrease in the quantitative value of histidine, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, an increase in the quantitative value of phenylalanine, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, an increase in the quantitative value of tyrosine, when compared to the control sample or to the control value, may be indicative of the subject having an increased risk of developing the circulatory disease, such as a multiple valve disease, pulmonary embolism, a nonrheumatic aortic valve disorder, atrial fibrillation and/or flutter.

In an embodiment, a risk score defined as $\beta_0 + \beta_1^*$ concentration (glycoprotein acetyls) $+ \beta_2^*$ concentration (albumin), where $\beta_0$ is an intercept term, $\beta_1$ is the weighted coefficient attributed to the concentration of glycoprotein acetyls, and $\beta_2$ is the weighted coefficient attributed to the concentration of albumin, may be indicative of the subject having an increased risk of developing the circulatory disease, such as a multiple valve disease, pulmonary embolism, a nonrheumatic aortic valve disorder, atrial fibrillation and/or flutter.

In an embodiment, a risk score defined as $\beta_0 + \beta_1^*$ concentration (glycoprotein acetyls) $+ \beta_2^*$ concentration (fatty acid measure), where $\beta_0$ is an intercept term, $\beta_1$ is the weighted coefficient attributed to the concentration of glycoprotein acetyls, $\beta_2$ is the weighted coefficient attributed to the fatty acid measure, may be indicative of the subject having an increased risk of developing the circulatory disease, such as a multiple valve disease, pulmonary embolism, a nonrheumatic aortic valve disorder, atrial fibrillation and/or flutter. The fatty acid measure may be one or more of the following fatty acids or their ratio to total fatty acids: linoleic acid, omega-6 fatty acids, saturated fatty acids and/or fatty acid degree of unsaturation.

In an embodiment, a risk score defined as $\beta_0 + \beta_1^*$ concentration (glycoprotein acetyls) $+ \beta_2^*$ concentration (albumin) $+ \beta_3^*$ concentration (fatty acid measure), where $\beta_0$ is an intercept term, $\beta_1$ is the weighted coefficient attributed to the concentration of glycoprotein acetyls, $\beta_2$ is the weighted coefficient attributed to the concentration of albumin, and $\beta_3$ is the weighted coefficient attributed to the concentration of the fatty acid measure may be indicative of the subject having an increased risk of developing the circulatory disease, such as a multiple valve disease, pulmonary embolism, a nonrheumatic aortic valve disorder, atrial fibrillation and/or flutter. The fatty acid measure may be one or more of the following fatty acids or their ratio to total fatty acids: linoleic acid, omega-6 fatty acids, saturated fatty acids and/or fatty acid degree of unsaturation.

The term "combination" may, at least in some embodiments, be understood such that the method comprises using a risk score, hazard ratio, odds ratio, and/or predicted absolute risk or relative risk calculated on the basis of the quantitative value(s) of the biomarkers. For example, if quantitative values of both glycoprotein acetyls and albumin are determined, the quantitative values of both biomarkers may be compared to the control sample or the control value separately, or a risk score, hazard ratio, odds ratio, and/or predicted absolute risk or relative risk calculated on the basis of the quantitative value(s) of both the biomarkers, and the risk score, hazard ratio, odds ratio, and/or predicted absolute risk or relative risk may be compared to the control sample or the control value.

In an embodiment, the method may comprise determining in the biological sample obtained from the subject a quantitative value of the following biomarkers:

- glycoprotein acetyls;
- albumin; and

comparing the quantitative value(s) of the biomarkers and/or a combination thereof to a control sample or to a control value(s);
wherein an increase or a decrease in the quantitative value(s) of the biomarkers and/or the combination thereof, when compared to the control sample or to the control value, is/are indicative of the subject having an increased risk of developing the circulatory disease. An increase in the quantitative value of glycoprotein acetyls and a decrease in the quantitative value of albumin, when compared to the control sample or to the control value, may be indicative of the subject having an increased risk of developing the circulatory disease.

In an embodiment, the method may comprise determining in the biological sample obtained from the subject a quantitative value of the following biomarkers:

- glycoprotein acetyls,
- at least one fatty acid measure(s) of the following fatty acids or their ratio to total fatty acids: linoleic acid, omega-6 fatty acids, saturated fatty acids and/or fatty acid degree of unsaturation; and

comparing the quantitative value(s) of the biomarkers and/or a combination thereof to a control sample or to a control value(s);
wherein an increase or a decrease in the quantitative value(s) of the biomarkers and/or the combination thereof, when compared to the control sample or to the control value, is/are indicative of the subject having an increased risk of developing the circulatory disease. An increase in the quantitative value of glycoprotein acetyls and a decrease in the quantitative value of linoleic acid and/or omega-6 fatty acids and/or fatty acid degree of unsaturation and/or their ratio to total fatty acids, and/or an increase in the quantitative value of saturated fatty acids and/or its ratio to total fatty acids, when compared to the control sample or to the control value, may be indicative of the subject having an increased risk of developing the circulatory disease.

In an embodiment, the method may comprise determining in the biological sample obtained from the subject a quantitative value of the following biomarkers:

- albumin,
- at least one fatty acid measure(s) of the following fatty acids or their ratio to total fatty acids: linoleic acid, omega-6 fatty acids, saturated fatty acids and/or fatty acid degree of unsaturation; and

comparing the quantitative value(s) of the biomarkers and/or a combination thereof to a control sample or to a control value(s);
wherein an increase or a decrease in the quantitative value(s) of the biomarkers and/or the combination thereof, when compared to the control sample or to the control value, is/are indicative of the subject having an increased risk of developing the circulatory disease. A decrease in the quantitative value of albumin and a decrease in the quantitative value of linoleic acid and/or omega-6 fatty acids and/or fatty acid degree of unsaturation and/or their ratio to total fatty acids, and/or an increase in the quantitative value of saturated fatty acids and/or its ratio to total fatty acids, when compared to the control sample or to the control value, may be indicative of the subject having an increased risk of developing the circulatory disease.

In an embodiment, the method may comprise determining in the biological sample obtained from the subject a quantitative value of the following biomarkers:

- glycoprotein acetyls,
- albumin,
- at least one fatty acid measure(s) of the following fatty acids or their ratio to total fatty acids: linoleic acid, omega-6 fatty acids, saturated fatty acids and/or fatty acid degree of unsaturation; and

comparing the quantitative value(s) of the biomarkers and/or a combination thereof to a control sample or to a control value(s);
wherein an increase or a decrease in the quantitative value(s) of the biomarkers and/or the combination thereof, when compared to the control sample or to the control value, is/are indicative of the subject having an increased risk of developing the circulatory disease. An increase in the quantitative value of glycoprotein acetyls, a decrease in the quantitative value of albumin and a decrease in the quantitative value of linoleic acid and/or omega-6 fatty acids and/or fatty acid degree of unsaturation and/or their ratio to total fatty acids, and/or an increase in the quantitative value of saturated fatty acids and/or its ratio to total fatty acids, when compared to the control sample or to the control value, may be indicative of the subject having an increased risk of developing the circulatory disease.

**EXAMPLES**

Reference will now be made in detail to various embodiments, an example of which is illustrated in the accompanying drawings. The description below discloses some embodiments in such a detail that a person skilled in the art is able to utilize the embodiments based on the disclosure. Not all steps or features of the embodiments are discussed in detail, as many of the steps or features will be obvious for the person skilled in the art based on this specification.

**Abbreviations used in the Figures:**

LA%: Ratio of linoleic acid to total fatty acids
Omega-6 %: Ratio of omega-6 fatty acids to total fatty acids
SFA %: Ratio of saturated fatty acids to total fatty acids Unsaturation: Fatty acid degree of unsaturation
LA: Linoleic acid
Omega-6: Omega-6 fatty acids
IDL: Intermediate-density lipoprotein
LDL: Low-density lipoprotein
IDL-TG: Triglycerides in intermediate-density lipoprotein (IDL)
LDL-TG: Triglycerides in low-density lipoprotein (LDL)
CI: confidence interval
SD: standard deviation
BMI: Body mass index

**EXAMPLE 1**

Biomarker measures quantified by nuclear magnetic resonance (NMR) were investigated as to whether they could be predictive of a certain circulatory disease, such as multiple valve diseases, pulmonary embolism, nonrheumatic aortic valve disorders, atrial fibrillation and/or flutter. All analyses were conducted based on the UK Biobank, with approximately 115 000 study participants with blood biomarker data from NMR spectroscopy available.

**Study population**

Details of the design of the UK Biobank have been reported by Sudlow et al 2015, PLoS Med. 2015;12(3):e1001779. Briefly, UK Biobank recruited 502 639 participants aged 37-73 years in 22 assessment centres across the UK. All participants provided written informed consent and ethical approval was obtained from the North West Multi-Center Research Ethics Committee. Blood samples were drawn at baseline between 2007 and 2010. No selection criteria were applied to the sampling.

**Biomarker profiling**

From the entire UK Biobank population, a random subset of baseline plasma samples from 118 466 individuals were measured using the Nightingale NMR biomarker platform (Nightingale Health Ltd, Finland). This blood analysis method provides simultaneous quantification of many blood biomarkers, including lipoprotein lipids, circulating fatty acids, and various low-molecular weight metabolites including amino acids, ketone bodies and gluconeogenesis-related metabolites in molar concentration units. Technical details and epidemiological applications have been reviewed (Soininen et al 2015, Circ Cardiovasc Genet; 2015;8:192-206; Würtz et al 2017, Am J Epidemiol 2017;186:1084-1096). Values outside four interquartile ranges from median were considered as outliers and excluded.

**Epidemiological analyses of biomarker relations with the risk of a certain circulatory disease**

The blood biomarker associations with the risk for a certain circulatory disease were conducted based on UK Biobank data. Analyses focused on the relation of the biomarkers to the occurrence of a certain circulatory disease after the blood samples were collected, to determine if the individual biomarkers associate with the risk for future development of a certain circulatory disease. Examples using multi-biomarker scores, in the form weighted sums of biomarkers, were also explored to see if they could be predictive even more strongly than each individual biomarker.
Information on the disease events occurring after the blood samplings for all study participants were recorded from UK Hospital Episode Statistics data and death registries. All analyses are based on first occurrence of diagnosis, so that individuals with recorded diagnosis of the given disease prior to blood sampling were omitted from the statistical analyses. A composite endpoint of Certain Circulatory Diseases was defined based on any incident occurrence of ICD-10 diagnoses I08, I10, I26, I27, I31, I35, I42, I44, I45, I46, I48, I71, I74, I77, I80 or I89. More refined subtypes of the certain circulatory diseases were defined according to the ICD-10 diagnoses listed in Table 1.

The registry-based follow-up was from blood sampling in 2007-2010 through to 2020 (approximately 1 100 000 person-years). Specific diseases which had <150 disease events recorded during follow-up were left out of scope.

For biomarker association testing, Cox proportional-hazard regression models adjusted for age, sex, and UK Biobank assessment centre were used. Results were plotted in magnitudes per standard deviation of each biomarker measure to allow direct comparison of association magnitudes.

**Summary of results**

Baseline characteristics of the study population for biomarker analyses vs future risk of a certain circulatory disease are shown in Table 1. The number of incident disease events occurring after the blood sampling is listed for all the conditions analysed.

**Table 1: Clinical characteristics of study participants and the number of incident disease events analysed.**

| | |
|---|---|
| Total number of individuals with blood samples analysed | 118 456 |
| Study setting | Population sample of study volunteers from the UK |
| Percentage of women | 54.1% |
| Age range (years) | 39 – 71 |
| Median age (years) | 58 |
| Median BMI (kg/m2) | 26.8 |
| Follow-up time for disease events after blood sampling | 10-14 years |
| Diseases with similar biomarker relations | Number of individuals who developed the specified disease after the blood sampling |
| Certain Circulatory Diseases: any incident occurrence of ICD-10 codes I08, I10, I26, I27, I31, I35, I42, I44, I45, I46, I48, I71, I74, I77, I80 or I89 | 27 090 |
| Specific Circulatory diseases (defined by 3-character ICD-10 codes) | |
| I08: Multiple valve diseases | 1 277 |
| I10: Essential (primary) hypertension | 23 489 |
| I26: Pulmonary embolism | 1 410 |
| I27: Other pulmonary heart diseases | 462 |

| | |
|---|---|
| I31: Other diseases of pericardium | 518 |
| I35: Nonrheumatic aortic valve disorders | 1 269 |
| I42: Cardiomyopathy | 526 |
| I44: Atrioventricular and left bundle-branch block | 1 975 |
| I45: Other conduction disorders | 1 146 |
| I46: Cardiac arrest | 568 |
| I48: Atrial fibrillation and flutter | 5 861 |
| I71: Aortic aneurysm and dissection | 709 |
| I74: Arterial embolism and thrombosis | 311 |
| I77: Other disorders of arteries and arterioles | 508 |
| I80: Phlebitis and thrombophlebitis | 1 090 |
| I89: Other noninfective disorders of lymphatic vessels and lymph nodes | 410 |

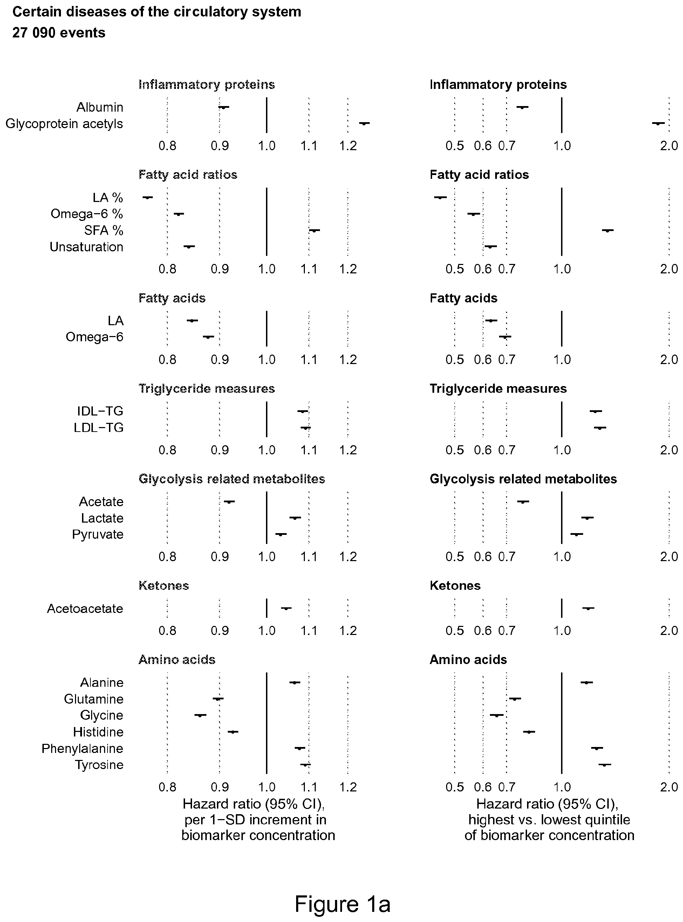

**Figure 1a** shows the hazard ratios for the 20 blood biomarkers with the future risk of Certain Circulatory Diseases (defined as a composite endpoint of the following circulatory disease ICD-10 diagnoses: I08, I10, I26, I27, I31, I35, I42, I44, I45, I46, I48, I71, I74, I77, I80 and I89; here termed "Certain Circulatory Diseases"). The left-hand side of the figure shows the hazard ratios when the biomarkers are analysed in absolute concentrations, scaled to standard deviations of the study population. The right-hand side shows the corresponding hazard ratios when individuals in the highest quintile of the biomarker concentration are compared to those in the lowest quintile. The results are based on statistical analyses of over 115 000 individuals from the UK Biobank, out of whom 27 090 developed a circulatory disease (one of the following circulatory disease ICD-10 diagnoses in the hospital registries or in the death records: I08, I10, I26, I27, I31, I35, I42, I44, I45, I46, I48, I71, I74, I77, I80 and I89) during approximately 10 years of follow-up. The analyses were adjusted for age, sex, and UK Biobank assessment centre in Cox proportional-hazard regression models. P-values were P<0.0001 (corresponding to multiple testing correction) for all associations. These results demonstrate that the 20 individual biomarkers are predictive of the risk for certain circulatory diseases in general population settings.

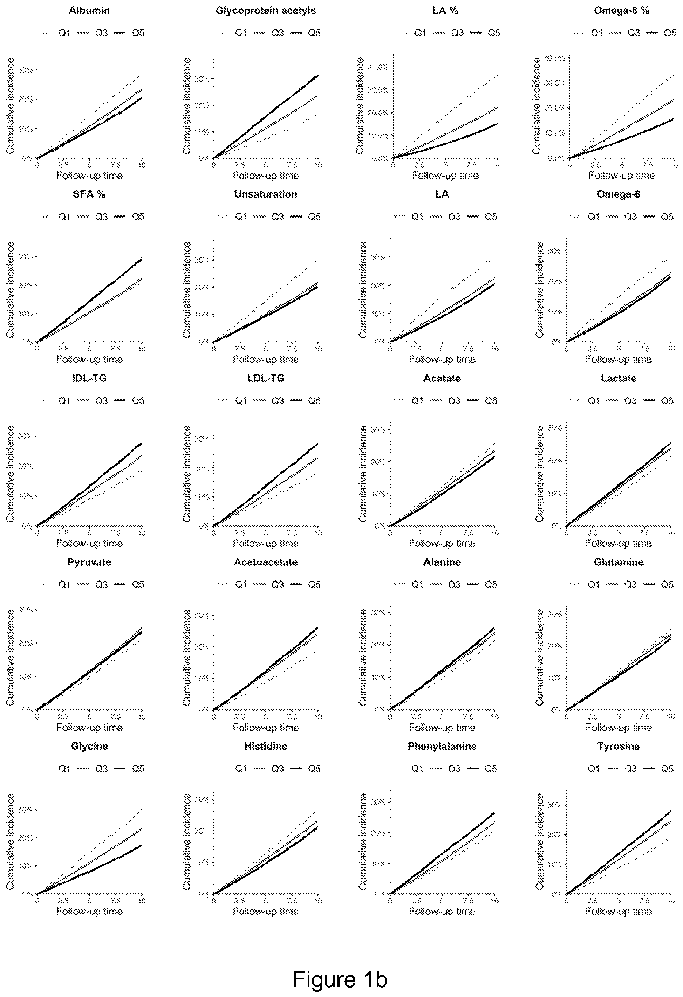

**Figure 1b** shows the Kaplan-Meier plots of the cumulative risk for "Certain Circulatory Diseases" for each of the 20 blood biomarkers according to the lowest, middle, and highest quintiles of biomarker concentrations. The results are based on statistical analyses of over 115 000 individuals from the UK Biobank, out of whom 27 090 developed a certain circulatory disease. These results further demonstrate that the 20 individual biomarkers are predictive of the risk for certain circulatory diseases in general population settings.

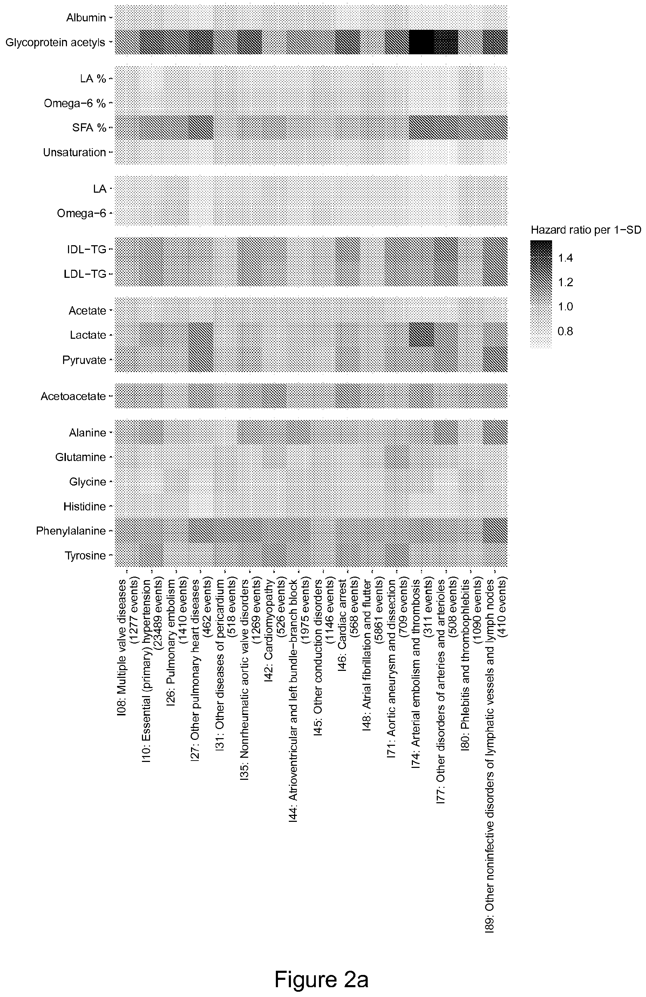

**Figure 2a** shows the hazard ratios of the 20 blood biomarkers for future onset of 16 specific circulatory diseases, defined by 3-character ICD-10 diagnosis codes. The results illustrate that the pattern of biomarker associations is highly consistent for all the 17 specific disorders.

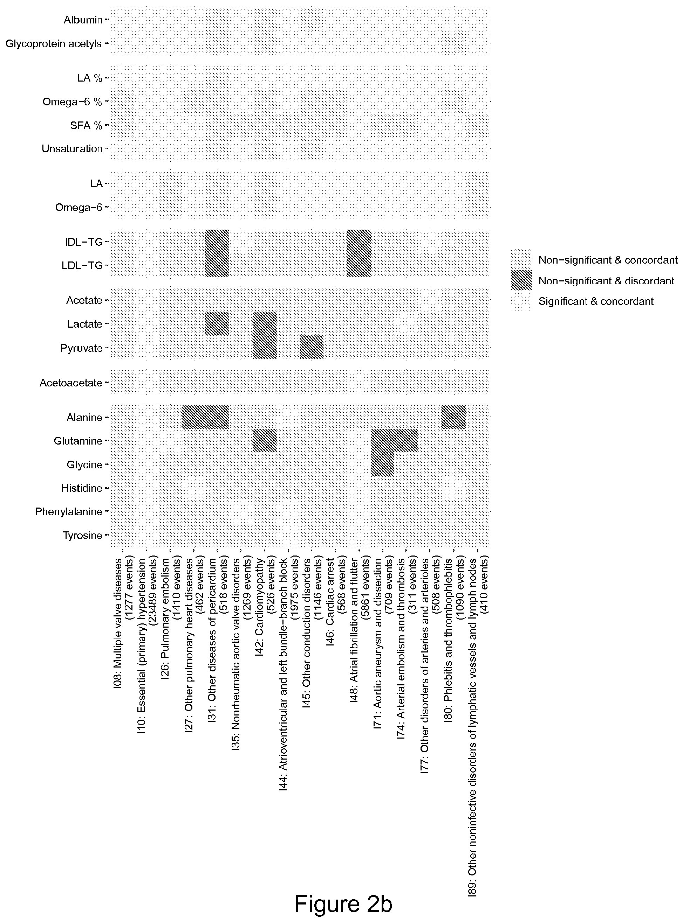

**Figure 2b** shows the consistency of the biomarker associations with the 16 specific circulatory diseases (defined by 3-character ICD-10 diagnosis codes) compared to the "Certain Circulatory Diseases" definition. Generally, the biomarker associations are all in the same direction of association as for "Certain Circulatory Diseases" or not statistically significant in the discordant direction. Any biomarker combination that strongly predicts "Certain Circulatory Diseases" will therefore also be predictive of all the listed specific circulatory diseases.

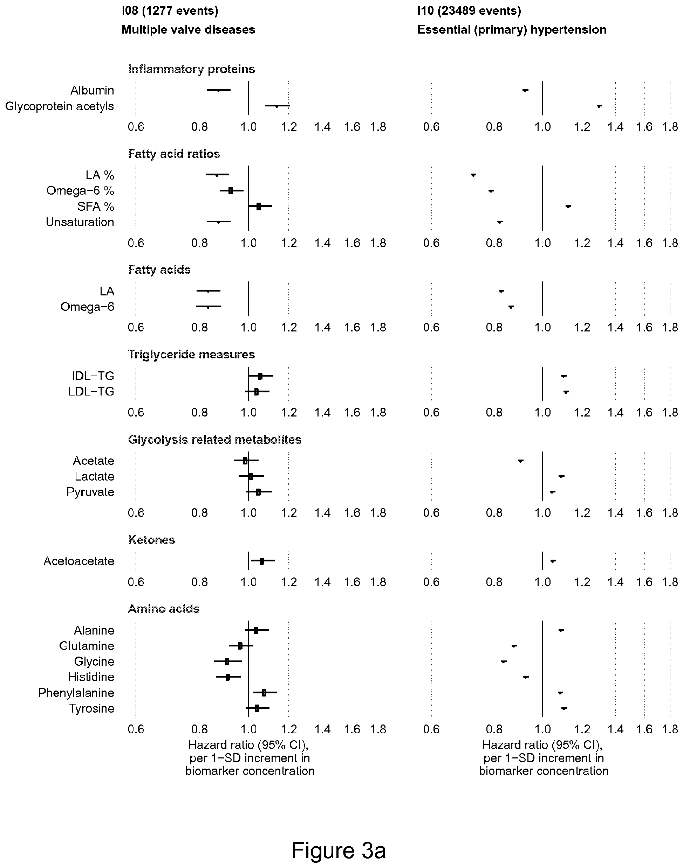

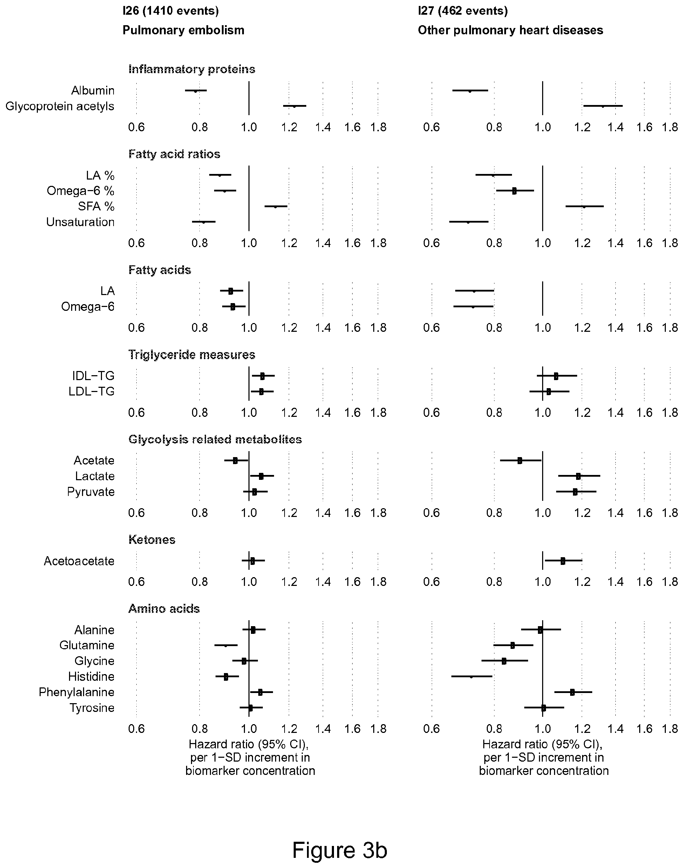

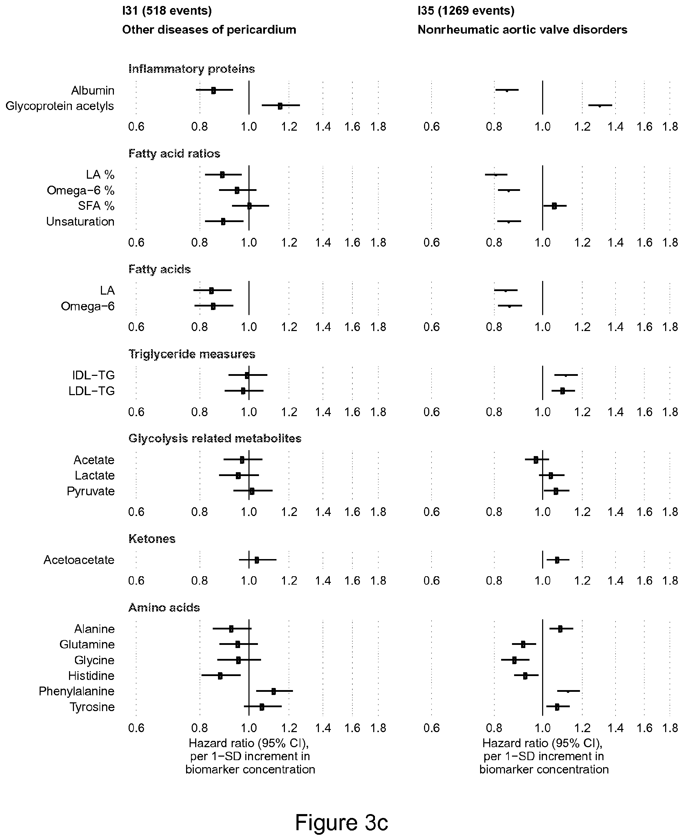

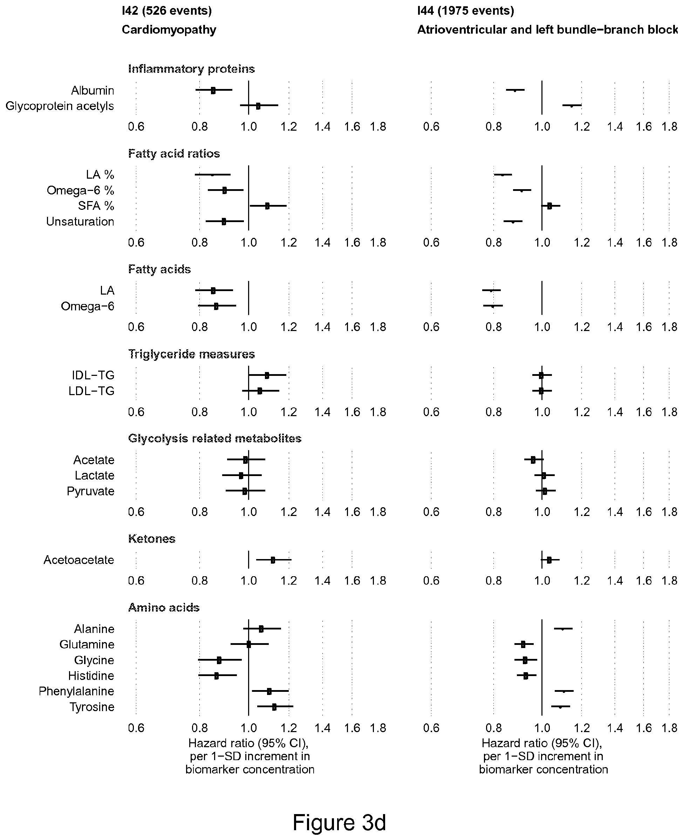

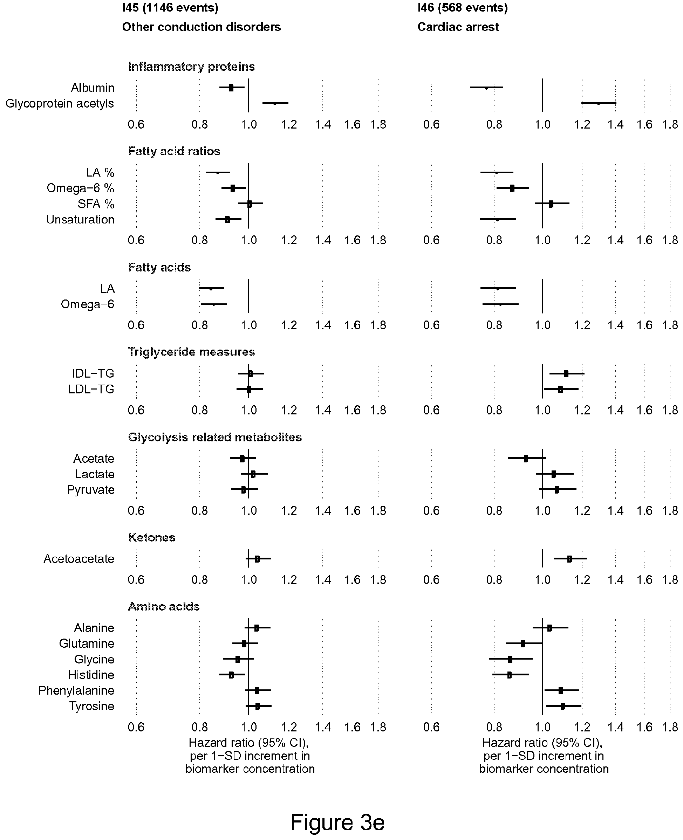

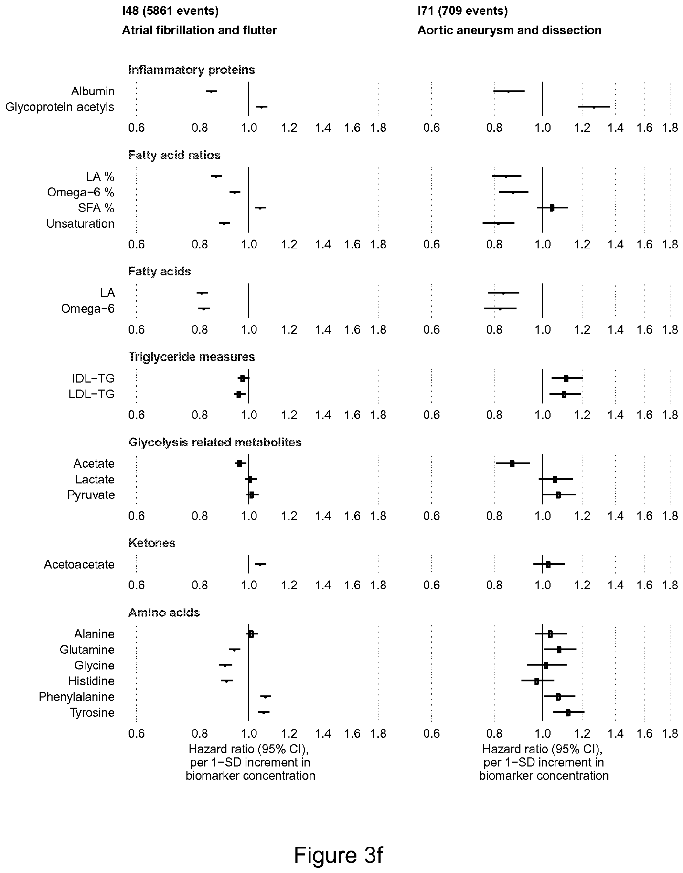

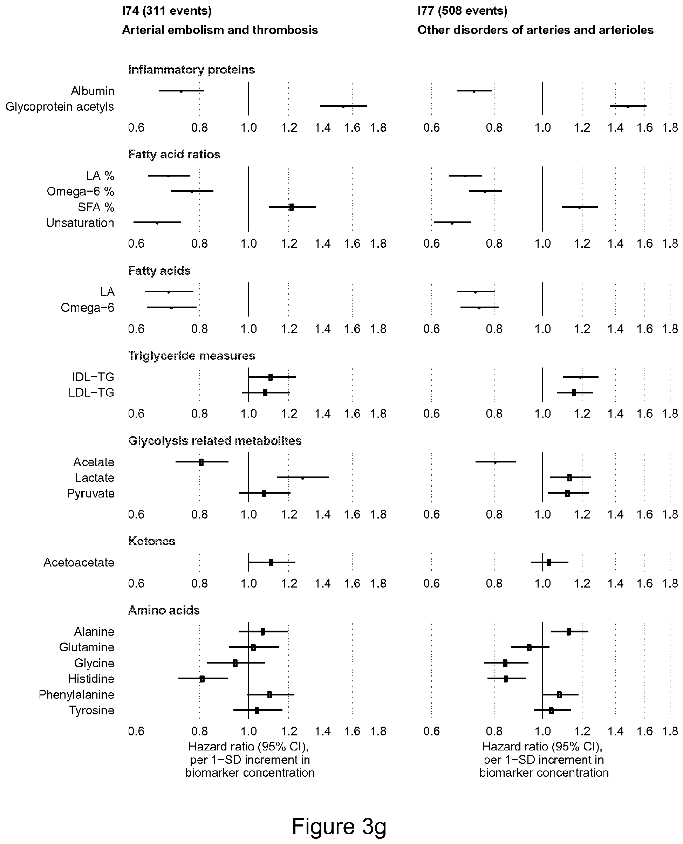

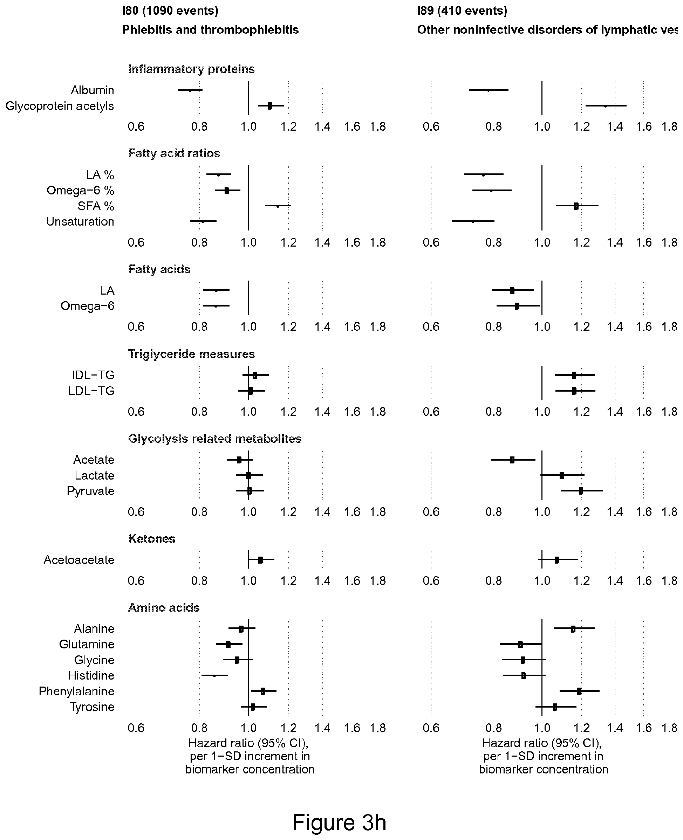

**Figures 3a-h** show the hazard ratios for the 20 blood biomarkers with future onset of each of the 16 specific circulatory diseases (defined by ICD-10 3-character diagnosis codes) studied here. The hazard ratios are shown in absolute concentrations, scaled to the standard deviation of each biomarker. The results are based on statistical analyses of over 115 000 individuals from the UK Biobank; the number of individuals who developed the specific disease during approximately 10 years of follow-up is indicated on the top of each plot. Filled circles denote that the P-value for association was P<0.0001 (corresponding to multiple testing correction), and open circles denote that the P-value for association was P $\geq$ 0.0001. The analyses were adjusted for age, sex, and UK Biobank assessment centre using Cox proportional-hazard regression models.

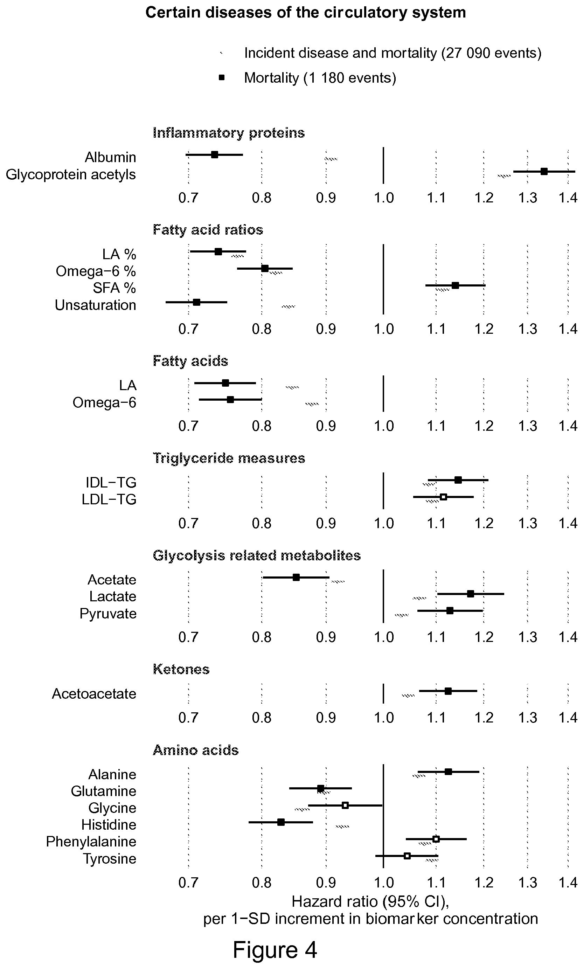

**Figure 4** shows hazard ratios for the 20 blood biomarkers with death from 'Certain Circulatory Diseases' (in black). For comparison are also shown the hazard ratios for incidence of Certain Circulatory Diseases (i.e. combined non-fatal and fatal events, in gray). The hazard ratios are shown for biomarkers analysed in absolute concentrations, scaled to standard deviations of the study population. These results demonstrate that the biomarkers are often stronger predictors of fatal circulatory disease events. The results were similar for the biomarker associations with fatal events due to specific circulatory diseases defined by 3-character ICD-10 diagnosis codes.

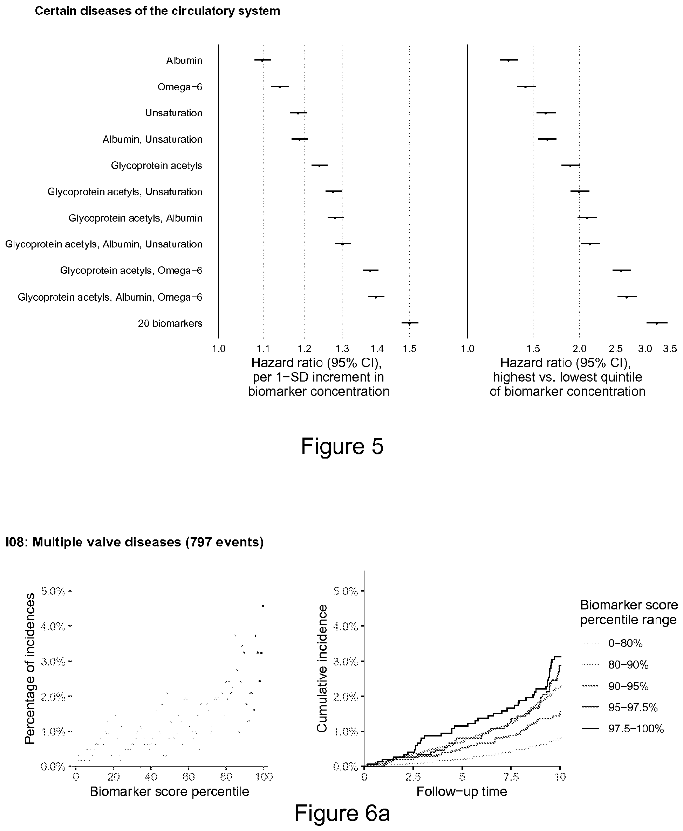

**Figure 5** shows examples of stronger association results with "Certain Circulatory Diseases" when two or more biomarkers are combined. The hazard ratios with the future risk of "Certain Circulatory Diseases" are shown for selected combinations of pairs of biomarkers, and examples of multi-biomarker scores. The results were similar with many other combinations, in particular inclusion of different fatty acid measures in addition to albumin and glycoprotein acetyls. The multi-biomarker scores are combined in the form of $\sum_i [\![ \beta_i * c_i ]\!] + \beta_0$ ; where $i$ is the index of summation over individual biomarkers, $\beta_i$ is the weighted coefficient attributed to biomarker $i$, $c_i$ is the blood concentration of biomarker $i$ and $\beta_0$ is an intercept term. $\beta_i$ multipliers are defined according to the multivariate association magnitude with the risk for "Certain Circulatory Diseases", examined in the statistical analyses of the UK Biobank study for the respective combination of biomarkers. The enhancements in association magnitudes were similar for the 17 specific types of circulatory diseases listed in Table 1 as those shown here.

**Illustrations of intended use: multi-biomarker scores for the risk prediction of certain circulatory diseases**

For illustration of intended applications related to the prediction of certain circulatory diseases, further epidemiological analyses are illustrated below. These applications are exemplified for the prediction of the risk for a multiple valve disease, a pulmonary embolism, a nonrheumatic aortic valve disorder, atrial fibrillation and/or flutter. Similar results apply to the other circulatory diseases listed in Table 1. Results are shown for a multi-biomarker score combining the 20 biomarkers featured in Figures 1-5. Similar results, albeit slightly weaker, are obtained with combinations of only two or three individual biomarkers.

**Figure 6a** shows the increase in risk for multiple valve diseases (ICD-10 code I08) along with increasing levels of a multi-biomarker score composed of the weighted sum of 20 biomarkers. On the left-hand side, the risk increase is plotted in the form of gradient percentile plots, showing the proportion of individuals who developed multiple valve diseases during follow-up when binning individuals into the percentiles of the biomarker levels. Each dot corresponds to approximately 500 individuals. In the Kaplan-Meier plots on the right-hand side, we illustrate the cumulative risk for multiple valve diseases during follow-up for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included for derivation of the multi-biomarker scores (n = 63 597).

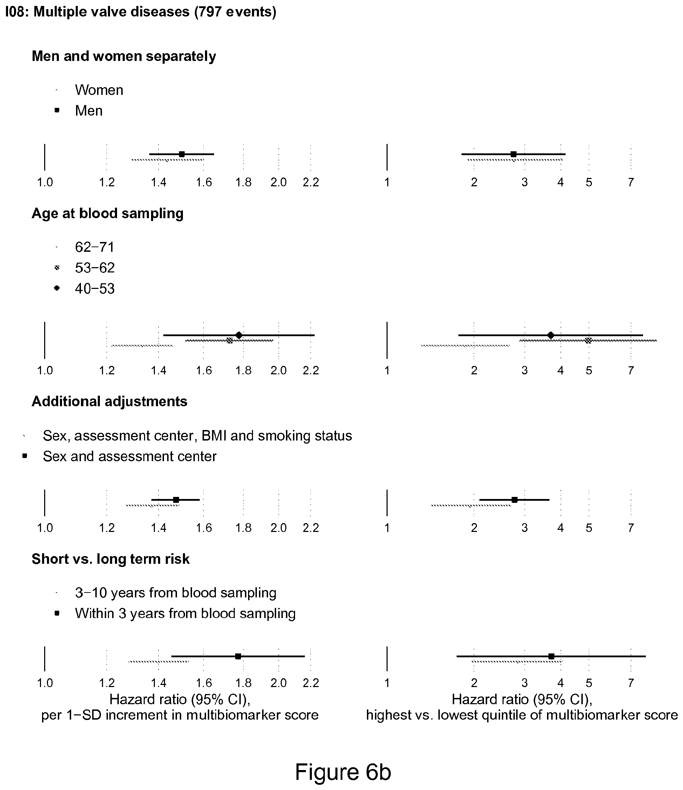

**Figure 6b** shows the hazard ratio of the same multi-biomarker score with future onset of multiple valve diseases (ICD-10 code I08) when accounting for relevant risk factor characteristics of the study participants. The first two panels demonstrate that the risk prediction works effectively for both men and women as well as for people at different ages at time of the blood sampling. The third panel shows that the magnitude of the hazard ratio is only modestly attenuated when accounting for body mass index and smoking status in the statistical modelling. The last panel shows that the results are substantially stronger for short term risk prediction.

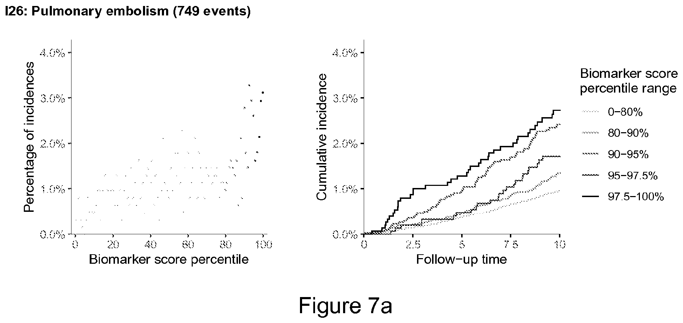

**Figure 7a** shows the increase in risk for pulmonary embolism (ICD-10 code I26) along with increasing levels of a multi-biomarker score composed of the weighted sum of 20 biomarkers. On the left-hand side, the risk increase is plotted in the form of gradient percentile plots, showing the proportion of individuals who developed pulmonary embolism during follow-up when binning individuals into the percentiles of the biomarker levels. Each dot corresponds to approximately 500 individuals. In the Kaplan-Meier plots on the right-hand side, we illustrate the cumulative risk for pulmonary embolism during follow-up for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included for derivation of the multi-biomarker scores (n = 63 392).

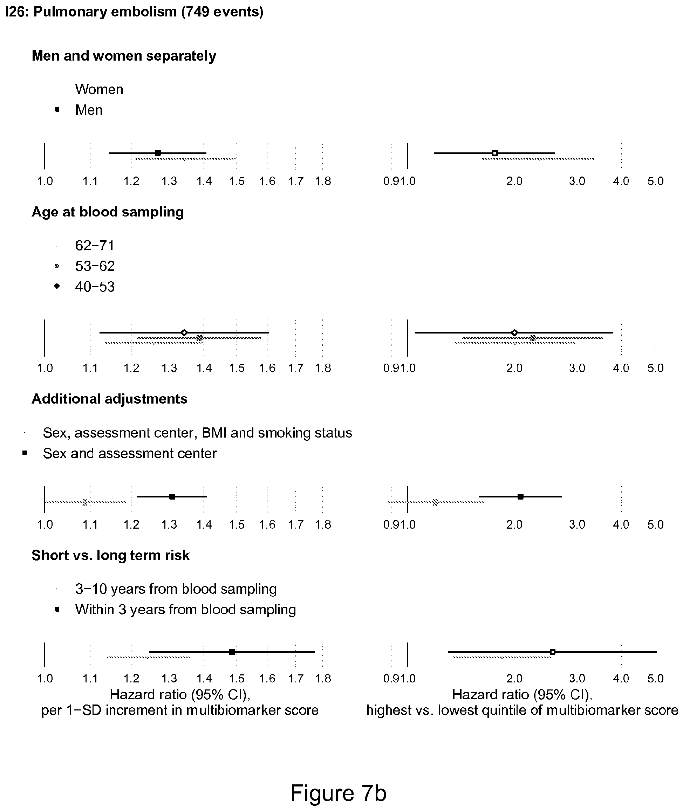

**Figure 7b** shows the hazard ratio of the same multi-biomarker score with future onset of pulmonary embolism (ICD-10 code I26) when accounting for relevant risk factor characteristics of the study participants. The first two panels demonstrate that the risk prediction works effectively for both men and women as well as for people at different ages at time of the blood sampling. The third panel shows that the magnitude of the hazard ratio is only modestly attenuated when accounting for body mass index and smoking status in the statistical modelling. The last panel shows that the results are substantially stronger for short term risk prediction.

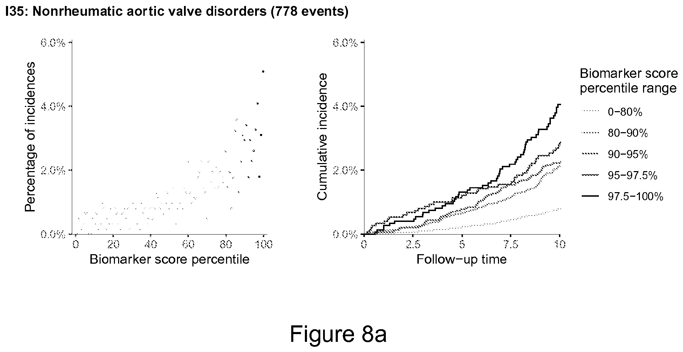

**Figure 8a** shows the increase in risk for nonrheumatic aortic valve disorders (ICD-10 code I35) along with increasing levels of a multi-biomarker score composed of the weighted sum of 20 biomarkers. On the left-hand side, the risk increase is plotted in the form of gradient percentile plots, showing the proportion of individuals who developed nonrheumatic aortic valve disorders during follow-up when binning individuals into the percentiles of the biomarker levels. Each dot corresponds to approximately 500 individuals. In the Kaplan-Meier plots on the right-hand side, we illustrate the cumulative risk for nonrheumatic aortic valve disorders during follow-up for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included for derivation of the multi-biomarker scores (n = 63 512).

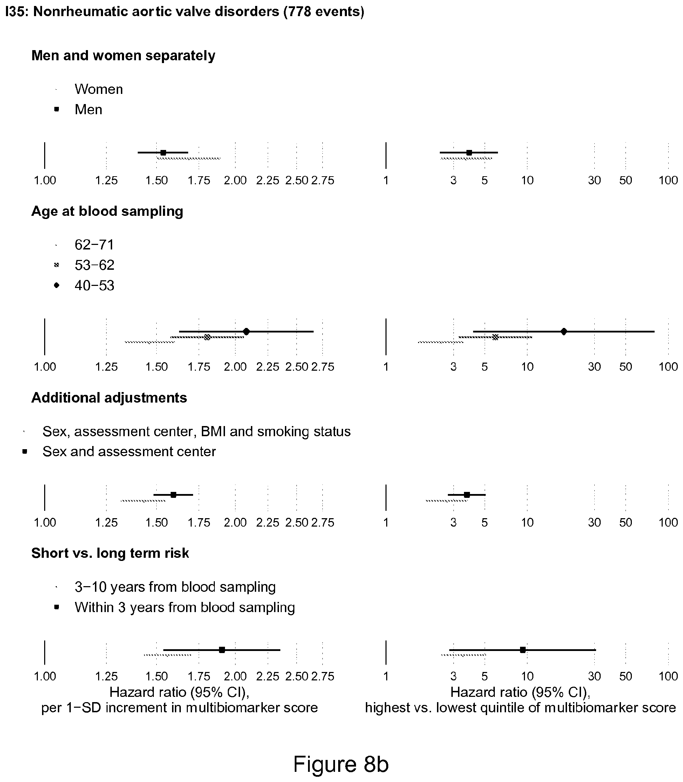

**Figure 8b** shows the hazard ratio of the same multi-biomarker score with future onset of nonrheumatic aortic valve disorders (ICD-10 code I35) when accounting for relevant risk factor characteristics of the study participants. The first two panels demonstrate that the risk prediction works effectively for both men and women as well as for people at different ages at time of the blood sampling. The third panel shows that the magnitude of the hazard ratio is only modestly attenuated when accounting for body mass index and smoking status in the statistical modelling. The last panel shows that the results are substantially stronger for short term risk prediction.

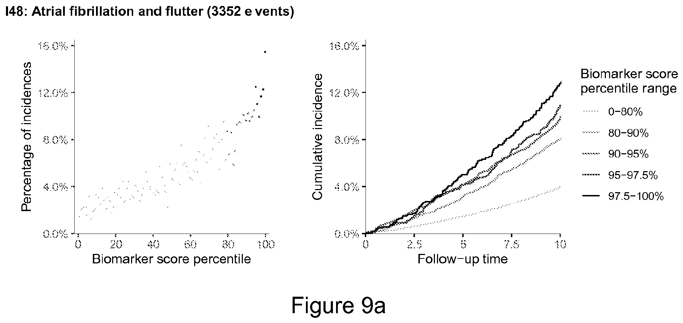

**Figure 9a** shows the increase in risk for atrial fibrillation and flutter (ICD-10 code I48) along with increasing levels of a multi-biomarker score composed of the weighted sum of 20 biomarkers. On the left-hand side, the risk increase is plotted in the form of gradient percentile plots, showing the proportion of individuals who developed atrial fibrillation and flutter during follow-up when binning individuals into the percentiles of the biomarker levels. Each dot corresponds to approximately 500 individuals. In the Kaplan-Meier plots on the right-hand side, we illustrate the cumulative risk for atrial fibrillation and flutter during follow-up for selected quantiles of the multi-biomarker score. Both plots serve to demonstrate that the risk is increasing non-linearly in the high end of the distribution of the multi-biomarker score. The plots are shown for the validation set part of the study population, i.e. 50% which was not included for derivation of the multi-biomarker scores (n = 62 756).

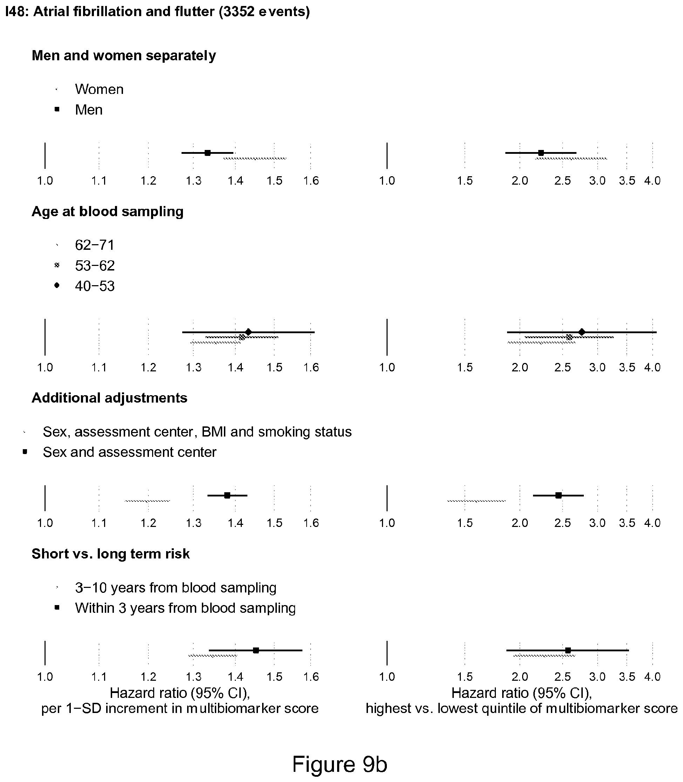

**Figure 9b** shows the hazard ratio of the same multi-biomarker score with future onset of atrial fibrillation and flutter (ICD-10 code I48) when accounting for relevant risk factor characteristics of the study participants. The first two panels demonstrate that the risk prediction works effectively for both men and women as well as for people at different ages at time of the blood sampling. The third panel shows that the magnitude of the hazard ratio is only modestly attenuated when accounting for body mass index and smoking status in the statistical modelling. The last panel shows that the results are substantially stronger for short term risk prediction.

It is obvious to a person skilled in the art that with the advancement of technology, the basic idea may be implemented in various ways. The embodiments are thus not limited to the examples described above; instead they may vary within the scope of the claims.

The embodiments described hereinbefore may be used in any combination with each other. Several of the embodiments may be combined together to form a further embodiment. A method disclosed herein may comprise at least one of the embodiments described hereinbefore. It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item refers to one or more of those items. The term "comprising" is used in this specification to mean including the feature(s) or act(s) followed thereafter, without excluding the presence of one or more additional features or acts.

**Claims**

1.   A method for determining whether a subject is at risk of developing a circulatory disease;

wherein the method comprises determining in a biological sample obtained from the subject a quantitative value of at least one biomarker of the following in the biological sample:

- glycoprotein acetyls,
- albumin,
- a ratio of linoleic acid to total fatty acids,
- a ratio of omega-6 fatty acids to total fatty acids,
- a ratio of saturated fatty acids to total fatty acids,
- fatty acid degree of unsaturation,
- linoleic acid,
- omega-6 fatty acids,
- triglycerides in intermediate-density lipoprotein (IDL),
- triglycerides in low-density lipoprotein (LDL),
- acetate,

- lactate,
- pyruvate,
- acetoacetate,
- alanine,
- glutamine,
- glycine,
- histidine,
- phenylalanine,
- tyrosine; and

comparing the quantitative value(s) of the at least one biomarker to a control sample or to a control value;
wherein an increase or a decrease in the quantitative value(s) of the at least one biomarker, when compared to the control sample or to the control value, is/are indicative of the subject having an increased risk of developing the circulatory disease;
wherein the circulatory disease comprises or is atrial fibrillation and/or flutter; and
wherein the at least one biomarker comprises or is glycoprotein acetyls, wherein glycoprotein acetyls refers to a nuclear magnetic resonance spectroscopy signal that represents the abundance of circulating glycated proteins.

2. The method according to claim 1, wherein the method comprises determining in the biological sample quantitative values of a plurality of the biomarkers, such as two, three, four, five or more of the biomarkers.

3. The method according to claim 1 or 2, wherein the method comprises determining in the biological sample obtained from the subject a quantitative value of the following biomarkers:

- glycoprotein acetyls;
- albumin; and
comparing the quantitative value(s) of the biomarkers to a control sample or to a control value(s);
wherein an increase or a decrease in the quantitative value(s) of the biomarkers, when compared to the control sample or to the control value, is/are indicative of the subject having an increased risk of developing the circulatory disease.

4. The method according to any one of claims 1 - 3, wherein the method comprises determining in the biological sample obtained from the subject a quantitative value of the following biomarkers:

- glycoprotein acetyls,
- at least one fatty acid measure(s) of the following: ratio of linoleic acid to total fatty acids, linoleic acid, ratio of omega-6 fatty acids to total fatty acids, omega-6 fatty acids, ratio of saturated fatty acids to total fatty acids, fatty acid degree of unsaturation; and
comparing the quantitative value(s) of the biomarkers to a control sample or to a control value(s);
wherein an increase or a decrease in the quantitative value(s) of the biomarkers, when compared to the control sample or to the control value, is/are indicative of the subject having an increased risk of developing the circulatory disease.

5. The method according to any one of claims 1 - 4, wherein the subject is generally healthy, or the subject has an already existing form of a circulatory disease and the risk of developing another or recurrent circulatory disease is determined.

6. The method according to any one of claims 1 - 5, wherein the subject is known not to suffer from a circulatory disease.

7. The method according to any one of claims 1 - 6, wherein the quantitative value of the at least one biomarker is/are measured using nuclear magnetic resonance spectroscopy.

8. The method according to any one of claims 1 - 7, wherein the method further comprises determining whether the subject is at risk of developing the circulatory disease using a risk score, hazard ratio, odds ratio, and/or predicted absolute risk or relative risk calculated on the basis of the quantitative value(s) of the at least one biomarker or of the plurality of the biomarkers.

9. The method according to claim 8, wherein the risk score, hazard ratio, odds ratio, and/or predicted relative risk and/or absolute risk may be calculated on the basis of at least one further measure, such as a characteristic of the subject.

10. The method according to claim 9, wherein the characteristic of the subject includes one or more of age, height, weight, body mass index, race or ethnic group, smoking, and/or family history of circulatory diseases.

11. The method according to any one of claims 1 - 10, wherein the method comprises determining in the biological sample obtained from the subject a quantitative value/values of the following biomarkers:

- glycoprotein acetyls,
- albumin,
- a ratio of linoleic acid to total fatty acids,
- a ratio of omega-6 fatty acids to total fatty acids,
- a ratio of saturated fatty acids to total fatty acids,
- fatty acid degree of unsaturation,
- linoleic acid,
- omega-6 fatty acids,
- triglycerides in intermediate-density lipoprotein (IDL),
- triglycerides in low-density lipoprotein (LDL),
- acetate,
- lactate,
- pyruvate,
- acetoacetate,
- alanine,
- glutamine,
- glycine,
- histidine,
- phenylalanine,
- tyrosine; and
comparing the quantitative value(s) of the biomarkers to a control sample or to a control value(s);
wherein an increase or a decrease in the quantitative value(s) of the biomarkers, when compared to the control sample or to the control value, is/are indicative of the subject having an increased risk of developing the circulatory disease.


**Patentansprüche**

1. Verfahren zur Bestimmung, ob bei einem Probanden das Risiko besteht, eine Kreislauferkrankung zu entwickeln;

wobei das Verfahren umfasst,
Bestimmen in einer vom Probanden entnommenen biologischen Probe eines quantitativen Werts mindestens eines der folgenden Biomarkern in der biologischen Probe:

- Glykoprotein-Acetyle,
- Albumin,
- ein Verhältnis von Linolsäure zu Gesamtfettsäuren,
- ein Verhältnis von Omega-6-Fettsäuren zu Gesamtfettsäuren,
- ein Verhältnis von gesättigten Fettsäuren zu Gesamtfettsäuren,
- Grad der Fettsäure-Ungesättigtheit,
- Linolsäure,
- Omega-6-Fettsäuren,
- Triglyceride in Lipoproteinen mittlerer Dichte (IDL),
- Triglyceride in Lipoproteinen niedriger Dichte (LDL),
- Acetat,
- Laktat,
- Pyruvat,
- Acetoacetat,
- Alanin,
- Glutamin,
- Glycin,
- Histidin,

- Phenylalanin,
- Tyrosin; und

Vergleichen des quantitativen Werts (der quantitativen Werte) des mindestens einen Biomarkers mit einer Kontrollprobe oder einem Kontrollwert;

wobei eine Zunahme oder eine Abnahme des quantitativen Werts (der quantitativen Werte) des mindestens einen Biomarkers im Vergleich zur Kontrollprobe oder zum Kontrollwert darauf hinweist, dass der Proband ein erhöhtes Risiko für die Entwicklung der Kreislauferkrankung aufweist;

wobei die Kreislauferkrankung Vorhofflimmern und/oder Vorhofflattern umfasst oder ist; und

wobei der mindestens eine Biomarker Glykoprotein-Acetyle umfasst oder ist, wobei Glykoprotein-Acetyle ein Kernspinresonanzspektroskopiesignal bezeichnen, das die Häufigkeit zirkulierender glykosylierter Proteine darstellt.

2. Verfahren nach Anspruch 1, wobei das Verfahren das Bestimmen quantitativer Werte einer Vielzahl der Biomarker, beispielsweise zwei, drei, vier, fünf oder mehr der Biomarker, in der biologischen Probe umfasst.

3. Verfahren nach Anspruch 1 oder 2, wobei das Verfahren umfasst

Bestimmen eines quantitativen Werts der folgenden Biomarker in der von dem Probanden erhaltenen biologischen Probe:

- Glykoprotein-Acetyle;
- Albumin; und

Vergleichen des quantitativen Werts (der quantitativen Werte) der Biomarker mit einer Kontrollprobe oder einem Kontrollwert (Kontrollwerten);

wobei eine Zunahme oder Abnahme des quantitativen Werts (der quantitativen Werte) der Biomarker im Vergleich zur Kontrollprobe oder zum Kontrollwert darauf hinweist, dass der Proband ein erhöhtes Risiko für die Entwicklung der Kreislauferkrankung aufweist.

4. Verfahren nach einem der Ansprüche 1 bis 3, wobei das Verfahren umfasst:

Bestimmen eines quantitativen Werts der folgenden Biomarker in der von dem Probanden erhaltenen biologischen Probe:

- Glykoprotein-Acetyle,
- mindestens einen Fettsäurewert aus den Folgenden: Verhältnis von Linolsäure zu Gesamtfettsäuren, Linolsäure, Verhältnis von Omega-6-Fettsäuren zu Gesamtfettsäuren, Omega-6-Fettsäuren, Verhältnis von gesättigten Fettsäuren zu Gesamtfettsäuren, Fettsäure-Ungesättigtheitsgrad; und

Vergleichen des quantitativen Werts (der quantitativen Werte) der Biomarker mit einer Kontrollprobe oder einem Kontrollwert (Kontrollwerten);

wobei eine Zunahme oder Abnahme des quantitativen Werts (der quantitativen Werte) der Biomarker im Vergleich zur Kontrollprobe oder zum Kontrollwert darauf hinweist, dass der Proband ein erhöhtes Risiko für die Entwicklung der Kreislauferkrankung aufweist.

5. Verfahren nach einem der Ansprüche 1 bis 4, wobei der Proband allgemein gesund ist oder der Proband bereits eine Form einer Kreislauferkrankung aufweist und das Risiko für die Entwicklung einer weiteren oder wiederkehrenden Kreislauferkrankung bestimmt wird.

6. Verfahren nach einem der Ansprüche 1 bis 5, wobei bekannt ist, dass der Proband nicht an einer Kreislauferkrankung leidet.

7. Verfahren nach einem der Ansprüche 1 bis 6, wobei der quantitative Wert des mindestens einen Biomarkers mittels Kernspinresonanzspektroskopie gemessen wird.

8. Verfahren nach einem der Ansprüche 1 bis 7, wobei das Verfahren ferner umfasst,
Bestimmen, ob bei dem Probanden ein Risiko für die Entwicklung der Kreislauferkrankung besteht, wobei ein Risikowert, eine Gefährdungsquote, ein Chancenverhältnis und/oder ein vorhergesagtes absolutes Risiko oder relatives Risiko verwendet wird, das/die auf der Grundlage des quantitativen Werts (der quantitativen Werte) des mindestens einen Biomarkers oder der Vielzahl von Biomarkern berechnet wird/werden.

9. Verfahren nach Anspruch 8, wobei der Risikowert, die Gefährdungsquote, das Chancenverhältnis und/oder das vorhergesagte relative Risiko und/oder das vorhergesagte absolute Risiko auf der Grundlage mindestens einer weiteren Messgröße, beispielsweise einer Charakteristik des Probanden, berechnet werden können.

10. Verfahren nach Anspruch 9, wobei die Charakteristik des Probanden eines oder mehrere der folgenden Merkmale umfasst: Alter, Größe, Gewicht, Body-Mass-Index, Rasse oder ethnische Zugehörigkeit, Rauchen und/oder familiäre Vorbelastung mit Kreislauferkrankungen.

11. Verfahren nach einem der Ansprüche 1 bis 10, wobei das Verfahren umfasst
Bestimmen eines quantitativen Werts (quantitativer Werte) der folgenden Biomarker in der von dem Probanden erhaltenen biologischen Probe:

- Glykoprotein-Acetyle,
- Albumin,
- ein Verhältnis von Linolsäure zu Gesamtfettsäuren,
- ein Verhältnis von Omega-6-Fettsäuren zu Gesamtfettsäuren,
- ein Verhältnis von gesättigten Fettsäuren zu Gesamtfettsäuren,
- Grad der Fettsäure-Ungesättigtheit,
- Linolsäure,
- Omega-6-Fettsäuren,
- Triglyceride in Lipoproteinen mittlerer Dichte (IDL),
- Triglyceride in Lipoproteinen niedriger Dichte (LDL),
- Acetat,
- Laktat,
- Pyruvat,
- Acetoacetat,
- Alanin,
- Glutamin,
- Glycin,
- Histidin,
- Phenylalanin,
- Tyrosin; und
Vergleichen des quantitativen Werts (der quantitativen Werte) der Biomarker mit einer Kontrollprobe oder einem Kontrollwert (Kontrollwerten);
wobei eine Zunahme oder eine Abnahme des quantitativen Werts (der quantitativen Werte) der Biomarker im Vergleich zur Kontrollprobe oder zum Kontrollwert darauf hinweist, dass der Proband ein erhöhtes Risiko für die Entwicklung der Kreislauferkrankung aufweist.

## Revendications

1. Procédé pour déterminer si un sujet est à risque de développer une maladie circulatoire ;

dans lequel le procédé comprend la détermination dans un échantillon biologique obtenu à partir du sujet d'une valeur quantitative d'au moins un biomarqueur parmi les suivants dans l'échantillon biologique :

- les acétyles de glycoprotéines,
- l'albumine,
- un rapport acide linoléique sur acides gras totaux,
- un rapport acides gras oméga-6 sur acides gras totaux,
- un rapport acides gras saturés sur acides gras totaux,
- le degré d'insaturation des acides gras,
- l'acide linoléique,
- les acides gras oméga-6,

- les triglycérides dans les lipoprotéines de densité intermédiaire (IDL),
- les triglycérides dans les lipoprotéines de basse densité (LDL),
- l'acétate,
- le lactate,
- le pyruvate,
- l'acétoacétate,
- l'alanine,
- la glutamine,
- la glycine,
- l'histidine,
- la phénylalanine,
- la tyrosine ; et

la comparaison de la (des) valeur(s) quantitative(s) de l'au moins un biomarqueur à un échantillon témoin ou à une valeur témoin ;
dans lequel une augmentation ou une diminution de la (des) valeur(s) quantitative(s) de l'au moins un biomarqueur, par rapport à l'échantillon témoin ou à la valeur témoin, indique que le sujet présente un risque accru de développer la maladie circulatoire ;
dans lequel la maladie circulatoire comprend ou est une fibrillation et/ou un flutter auriculaire ; et
dans lequel l'au moins un biomarqueur comprend ou est des acétyles de glycoprotéines, dans lequel les acétyles de glycoprotéines font référence à un signal de spectroscopie de résonance magnétique nucléaire qui représente l'abondance des protéines glyquées circulantes.

2. Procédé selon la revendication 1, dans lequel le procédé comprend la détermination dans l'échantillon biologique de valeurs quantitatives d'une pluralité de biomarqueurs, tels que deux, trois, quatre, cinq des biomarqueurs ou plus.

3. Procédé selon la revendication 1 ou la revendication 2, dans lequel le procédé comprend la détermination dans l'échantillon biologique obtenu à partir du sujet d'une valeur quantitative des biomarqueurs suivants :

- les acétyles de glycoprotéines ;
- l'albumine ; et
la comparaison de la (des) valeur(s) quantitative(s) des biomarqueurs à un échantillon témoin ou à une (des) valeur(s) témoin(s),
dans lequel une augmentation ou une diminution de la (des) valeur(s) quantitative(s) des biomarqueurs, par rapport à l'échantillon témoin ou à la valeur témoin, indique que le sujet présente un risque accru de développer la maladie circulatoire.

4. Procédé selon l'une quelconque des revendications 1 à 3, dans lequel le procédé comprend la détermination dans l'échantillon biologique obtenu à partir du sujet d'une valeur quantitative des biomarqueurs suivants :

- les acétyles de glycoprotéines,
- au moins une mesure d'acide gras parmi les suivantes : rapport acide linoléique sur acides gras totaux, acide linoléique, rapport acides gras oméga-6 sur acides gras totaux, acides gras oméga-6, rapport acides gras saturés sur acides gras totaux, degré d'insaturation des acides gras ; et
la comparaison de la (des) valeur(s) quantitative(s) des biomarqueurs à un échantillon témoin ou à une (des) valeur(s) témoin(s) ;
dans lequel une augmentation ou une diminution de la (des) valeur(s) quantitative(s) des biomarqueurs, par rapport à l'échantillon témoin ou à la valeur témoin, indique que le sujet présente un risque accru de développer la maladie circulatoire.

5. Procédé selon l'une quelconque des revendications 1 à 4, dans lequel le sujet est généralement en bonne santé, ou le sujet présente une forme déjà existante de maladie circulatoire et le risque de développer une autre maladie circulatoire ou une maladie circulatoire récurrente est déterminé.

6. Procédé selon l'une quelconque des revendications 1 à 5, dans lequel le sujet est connu pour ne pas souffrir d'une maladie circulatoire.

7. Procédé selon l'une quelconque des revendications 1 à 6, dans lequel la valeur quantitative de l'au moins un biomarqueur est mesurée par spectroscopie par résonance magnétique nucléaire.

8. Procédé selon l'une quelconque des revendications 1 à 7, dans lequel le procédé comprend en outre la détermination du fait que le sujet est ou non à risque de développer la maladie circulatoire en utilisant un score de risque, un rapport de risque, un rapport de cotes et/ou un risque absolu ou un risque relatif prédit calculé sur la base de la (des) valeur(s) quantitative(s) de l'au moins un biomarqueur ou de la pluralité des biomarqueurs.

9. Procédé selon la revendication 8, dans lequel le score de risque, le rapport de risque, le rapport de cotes et/ou le risque relatif et/ou risque absolu prédit peuvent être calculés sur la base d'au moins une mesure supplémentaire, telle qu'une caractéristique du sujet.

10. Procédé selon la revendication 9, dans lequel la caractéristique du sujet inclut un ou plusieurs parmi l'âge, la taille, le poids, l'indice de masse corporelle, la race ou le groupe ethnique, le tabagisme et/ou les antécédents familiaux de maladies circulatoires.

11. Procédé selon l'une quelconque des revendications 1 à 10, dans lequel le procédé comprend la détermination dans l'échantillon biologique obtenu à partir du sujet d'une ou plusieurs valeurs quantitatives des biomarqueurs suivants :

    - les acétyles de glycoprotéines,
    - l'albumine,
    - un rapport acide linoléique sur acides gras totaux,
    - un rapport acides gras oméga-6 sur acides gras totaux,
    - un rapport acides gras saturés sur acides gras totaux,
    - le degré d'insaturation des acides gras,
    - l'acide linoléique,
    - les acides gras oméga-6,
    - les triglycérides dans les lipoprotéines de densité intermédiaire (IDL),
    - les triglycérides dans les lipoprotéines de basse densité (LDL),
    - l'acétate,
    - le lactate,
    - le pyruvate,
    - l'acétoacétate,
    - l'alanine,
    - la glutamine,
    - la glycine,
    - l'histidine,
    - la phénylalanine,
    - la tyrosine ; et
la comparaison de la (des) valeur(s) quantitative(s) des biomarqueurs à un échantillon témoin ou à une (des) valeur(s) témoin(s),
dans lequel une augmentation ou une diminution de la (des) valeur(s) quantitative(s) des biomarqueurs, par rapport à l'échantillon témoin ou à la valeur témoin, indique que le sujet présente un risque accru de développer la maladie circulatoire.

**Certain diseases of the circulatory system**
**27 090 events**

Figure 1a

Figure 1b

Figure 2a

Figure 2b

Figure 3a

Figure 3b

Figure 3c

**I42 (526 events)**
**Cardiomyopathy**

**I44 (1975 events)**
**Atrioventricular and left bundle-branch block**

Inflammatory proteins

Albumin
Glycoprotein acetyls

0.6   0.8   1.0   1.2   1.4   1.6   1.8      0.6   0.8   1.0   1.2   1.4   1.6   1.8

Fatty acid ratios

LA %
Omega-6 %
SFA %
Unsaturation

0.6   0.8   1.0   1.2   1.4   1.6   1.8      0.6   0.8   1.0   1.2   1.4   1.6   1.8

Fatty acids

LA
Omega-6

0.6   0.8   1.0   1.2   1.4   1.6   1.8      0.6   0.8   1.0   1.2   1.4   1.6   1.8

Triglyceride measures

IDL-TG
LDL-TG

0.6   0.8   1.0   1.2   1.4   1.6   1.8      0.6   0.8   1.0   1.2   1.4   1.6   1.8

Glycolysis related metabolites

Acetate
Lactate
Pyruvate

0.6   0.8   1.0   1.2   1.4   1.6   1.8      0.6   0.8   1.0   1.2   1.4   1.6   1.8

Ketones

Acetoacetate

0.6   0.8   1.0   1.2   1.4   1.6   1.8      0.6   0.8   1.0   1.2   1.4   1.6   1.8

Amino acids

Alanine
Glutamine
Glycine
Histidine
Phenylalanine
Tyrosine

0.6   0.8   1.0   1.2   1.4   1.6   1.8      0.6   0.8   1.0   1.2   1.4   1.6   1.8
Hazard ratio (95% CI),
per 1-SD increment in
biomarker concentration

Hazard ratio (95% CI),
per 1-SD increment in
biomarker concentration

# Figure 3d

**I45 (1146 events)**
**Other conduction disorders**

**I46 (568 events)**
**Cardiac arrest**

Figure 3e

Figure 3f

I74 (311 events)
Arterial embolism and thrombosis

I77 (508 events)
Other disorders of arteries and arterioles

Inflammatory proteins

Albumin
Glycoprotein acetyls

0.6    0.8    1.0    1.2    1.4    1.6    1.8          0.6    0.8    1.0    1.2    1.4    1.6    1.8

Fatty acid ratios

LA %
Omega−6 %
SFA %
Unsaturation

0.6    0.8    1.0    1.2    1.4    1.6    1.8          0.6    0.8    1.0    1.2    1.4    1.6    1.8

Fatty acids

LA
Omega−6

0.6    0.8    1.0    1.2    1.4    1.6    1.8          0.6    0.8    1.0    1.2    1.4    1.6    1.8

Triglyceride measures

IDL−TG
LDL−TG

0.6    0.8    1.0    1.2    1.4    1.6    1.8          0.6    0.8    1.0    1.2    1.4    1.6    1.8

Glycolysis related metabolites

Acetate
Lactate
Pyruvate

0.6    0.8    1.0    1.2    1.4    1.6    1.8          0.6    0.8    1.0    1.2    1.4    1.6    1.8

Ketones

Acetoacetate

0.6    0.8    1.0    1.2    1.4    1.6    1.8          0.6    0.8    1.0    1.2    1.4    1.6    1.8

Amino acids

Alanine
Glutamine
Glycine
Histidine
Phenylalanine
Tyrosine

0.6    0.8    1.0    1.2    1.4    1.6    1.8          0.6    0.8    1.0    1.2    1.4    1.6    1.8

Hazard ratio (95% CI),
per 1−SD increment in
biomarker concentration

Hazard ratio (95% CI),
per 1−SD increment in
biomarker concentration

Figure 3g

Figure 3h

## Certain diseases of the circulatory system

˙ Incident disease and mortality (27 090 events)

■ Mortality (1 180 events)

Hazard ratio (95% CI),
per 1-SD increment in biomarker concentration

# Figure 4

**Certain diseases of the circulatory system**

Figure 5

**I08: Multiple valve diseases (797 events)**

Figure 6a

**I08: Multiple valve diseases (797 events)**

**Men and women separately**

- Women
- Men

**Age at blood sampling**

- 62–71
- 53–62
- 40–53

**Additional adjustments**

- Sex, assessment center, BMI and smoking status
- Sex and assessment center

**Short vs. long term risk**

- 3–10 years from blood sampling
- Within 3 years from blood sampling

Hazard ratio (95% CI),
per 1–SD increment in multibiomarker score

Hazard ratio (95% CI),
highest vs. lowest quintile of multibiomarker score

Figure 6b

**I26: Pulmonary embolism (749 events)**

Figure 7a

**I26: Pulmonary embolism (749 events)**

**Men and women separately**

· Women
■ Men

**Age at blood sampling**

· 62–71
✱ 53–62
◆ 40–53

**Additional adjustments**

· Sex, assessment center, BMI and smoking status
■ Sex and assessment center

**Short vs. long term risk**

· 3–10 years from blood sampling
■ Within 3 years from blood sampling

Hazard ratio (95% CI),
per 1–SD increment in multibiomarker score

Hazard ratio (95% CI),
highest vs. lowest quintile of multibiomarker score

Figure 7b

**I35: Nonrheumatic aortic valve disorders (778 events)**

Figure 8a

**I35: Nonrheumatic aortic valve disorders (778 events)**

**Men and women separately**

· Women
▪ Men

**Age at blood sampling**

· 62−71
※ 53−62
♦ 40−53

**Additional adjustments**

· Sex, assessment center, BMI and smoking status
▪ Sex and assessment center

**Short vs. long term risk**

· 3−10 years from blood sampling
▪ Within 3 years from blood sampling

Hazard ratio (95% CI),
per 1−SD increment in multibiomarker score

Hazard ratio (95% CI),
highest vs. lowest quintile of multibiomarker score

## Figure 8b

**I48: Atrial fibrillation and flutter (3352 e vents)**

Figure 9a

**I48: Atrial fibrillation and flutter (3352 events)**

**Men and women separately**

· Women
■ Men

**Age at blood sampling**

` 62-71
※ 53-62
♦ 40-53

**Additional adjustments**

` Sex, assessment center, BMI and smoking status
■ Sex and assessment center

**Short vs. long term risk**

· 3-10 years from blood sampling
■ Within 3 years from blood sampling

Hazard ratio (95% CI),
per 1-SD increment in multibiomarker score

Hazard ratio (95% CI),
highest vs. lowest quintile of multibiomarker score

# Figure 9b

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JANG et al.** Abstract 11299: The novel inflammatory marker Glyc a and incident atrial fibrillation in the multi-ethnic study of atherosclerosis. *Circulation*, 11 November 2019, 1-5
- **VIVIANO et al.** Proteomics of the epicardial fat secretome and its role in post-operative atrial fibrillation. *Europace*, 2018, vol. 20, 1201-1208
- **ADAMSSON ERYD et al.** Inflammation-sensitive proteins and risk of atrial fibrillation: a population-based cohort study. *European Journal of Epidemiology*, 2011, vol. 26, 449-455
- **MATTIOLI et al.** Atrial stunning, inflammation and nutritional status after cardioversion from atrial fibrillation. *International Journal of Cardiology*, 2008, vol. 129, 344-347
- **KETTUNEN et al.** *Nature Genetics*, 2012, vol. 44, 269-276
- **SOININEN et al.** *Circulation: Cardiovascular Genetics*, 2015, vol. 8, 212-206

- **RITCHIE et al.** *Cell Systems*, 2015, vol. 1 (4), 293-301
- **CONNELLY et al.** *J Transl Med.*, 2017, vol. 15 (1), 219
- **KETTUNEN et al.** *Circ Genom Precis Med.*, 2018, vol. 11, e002234
- **SOININEN et al.** *Analyst*, 2009, vol. 134, 1781-1785
- **WÜRTZ et al.** *American Journal of Epidemiology*, 2017, vol. 186 (9), 1084-1096
- **JULA et al.** *Arterioscler Thromb Vasc Biol*, 2005, vol. 25, 2152-2159
- **SUDLOW et al.** *PLoS Med.*, 2015, vol. 12 (3), e1001779
- **SOININEN et al.** *Circ Cardiovasc Genet;*, 2015, vol. 8, 192-206
- **WÜRTZ et al.** *Am J Epidemiol*, 2017, vol. 186, 1084-1096